# EUROPEAN PATENT SPECIFICATION

(11) **EP 2 914 273 B1**
(45) Date of publication and mention of the grant of the patent: **04.07.2018**
(21) Application number: 13849480.2
(22) Date of filing: 23.10.2013
(51) Int. Cl.: A61K 35/34, A61K 35/44, A61K 9/00, A61L 27/38

(54) **THERAPEUTIC CELLS DEPLETED OF SPECIFIC SUBPOPULATIONS OF CELLS FOR USE IN TISSUE REPAIR OF REGENERATION**
AN SPEZIFISCHEN ZELLSUBPOPULATIONEN ABGEREICHERTE THERAPEUTISCHE ZELLEN ZUR GEWEBEREPARATUR ODER -REGENERATION
CELLULES THÉRAPEUTIQUES APPAUVRIES EN SOUS-POPULATIONS SPÉCIFIQUES DE CELLULES DESTINÉES À ÊTRE UTILISÉES DANS LA RÉPARATION OU LA RÉGÉNÉRATION DE TISSU

(30) Priority: 26.10.2012 US 201261719324 P; 25.06.2013 US 201361839308 P
(43) Date of publication of application: 09.09.2015
(73) Proprietor: Cedars-Sinai Medical Center, Los Angeles, CA 90048 (US)
(72) Inventor: MARBAN, Eduardo, Los Angeles, CA 90048-1865 (US); CHENG, Ke, Los Angeles, CA 90048-1865 (US)
(74) Representative: Rees, Kerry
(86) International application number: PCT/US2013/066449
(87) International publication number: WO 2014/066545

(56) References cited:
- WO-A1-2012/135253
- WO-A2-2010/015665
- US-A1- 2010 040 587
- US-A1- 2010 303 909
- US-A1- 2012 039 857
- US-A1- 2012 253 102
- SMITH RACHEL RUCKDESCHEL ET AL: "Unselected Human Cardiosphere-derived Cells are Functionally Superior to c-Kit- or CD90-Purified Cardiosphere-derived Cells", CIRCULATION, vol. 118, no. 18, Suppl. 2, October 2008 (2008-10), page S420, XP9189994, & 81ST ANNUAL SCIENTIFIC SESSION OF THE AMERICAN-HEART-ASSOCIATION; NEW ORLEANS, LA, USA; NOVEMBER 08 -12, 2008 ISSN: 0009-7322
- SMITH RACHEL R ET AL: "Unique phenotype of cardiospheres derived from human endomyocardial biopsies", CIRCULATION, vol. 112, no. 17, Suppl. S, October 2005 (2005-10), pages U105-U106, XP9189993, & 78TH ANNUAL SCIENTIFIC SESSION OF THE AMERICAN-HEART-ASSOCIATION; DALLAS, TX, USA; NOVEMBER 13 -16, 2005 ISSN: 0009-7322
- K. CHENG ET AL: "Relative Roles of CD90 and c-Kit to the Regenerative Efficacy of Cardiosphere-Derived Cells in Humans and in a Mouse Model of Myocardial Infarction", JOURNAL OF THE AMERICAN HEART ASSOCIATION, vol. 3, no. 5, 9 October 2014 (2014-10-09) , pages e001260-e001260, XP055271592, ISSN: 2047-9980, DOI: 10.1161/JAHA.114.001260
- LI ET AL.: 'Direct Comparison of Different Stem Cell Types and Subpopulations Reveals Superior Paracrine Potency and Myocardial Repair Efficacy With Cardiosphere-Derived Cells' JOURNAL OF THE AMERICAN COLLEGE OF CARDIOLOGY vol. 59, no. 10, 06 March 2012, pages 942 - 953, XP028462740

## Description

### RELATED CASES

This application claims the benefit of U.S. Provisional Application No. 61/719,324, filed October 26, 2012 and U.S. Provisional Application No. 61/839,308, filed June 25, 2013.

### BACKGROUND

### Field

The present application relates generally to therapeutic cell populations, methods of preparing therapeutic populations of cells having specific subpopulations of cells removed, and compositions comprising therapeutic cell populations depleted of specific subpopulations for use in repair or regeneration of damaged or diseased tissue. In particular, several embodiments relate to methods of generating such depleted therapeutic cell populations and uses of same for tissue repair, tissue regeneration and/or functional tissue recovery such as, for example, treatment of damaged cardiac tissue after myocardial infarction. In one embodiment, the cells used are substantially depleted of c-kit. The cells used are substantially depleted of CD90. In one embodiment, the cells are substantially depleted of c-kit, CD90, and optionally one or more additional markers.

### Description of the Related Art

Stems cells are characterized by the ability to renew themselves through mitotic cell division and the ability to differentiate into a diverse range of specialized cell types. The two primary types of mammalian stem cells are embryonic stem cells and adult stem cells (e.g., non-embryonic stem cells). Embryonic stem cells are typically isolated from the inner cell mass of blastocysts and are pluripotent, meaning that the cells have the capacity to differentiate into all cell or tissue types. Adult stem cells are isolated from adult tissues and function as an ongoing repair system for adult organs.

Cardiovascular disease is currently the leading cause of death of both men and women in the United States, accounting for approximately 1 in every 3 deaths. Coronary heart disease is estimated to cost the United States $108.9 billion each year. A heart attack (or myocardial infarction, MI) occurs when one or more of the coronary arteries which supply oxygen-rich blood to the heart is blocked, denying oxygen to the heart. Myocardial infarction can result in ischemic death to the muscle and tissue of the heart and the formation of scar tissue. Following myocardial infarction, the function of the heart can be permanently impaired, potentially resulting in death or a reduced quality of life for the patient. According to the most recent statistics from the American Heart Association, an approximately 785,000 Americans suffer from a heart attack every year.

### SUMMARY

In several embodiments of the present invention, it has been discovered that cardiac cells depleted of c-kit offer a purer alternative to repairing and/or regenerating cardiac tissue. By depleting cells of all (or substantially all) cells that express c-kit, several embodiments of the invention provide unique several advantages. For example, in several embodiments, the invention comprises a therapeutic population of cells that, post-depletion, has about 1%, 5%, 10%, 20%, 25% (or ranges therein) of cells expressing a marker, such as c-kit, as compared to the non-depleted population (e.g., pre-depletion). As an example, a pre-depletion cell population may comprise 5% of cells that express c-kit, while the post-depletion cell population comprises 1.25% of such cells or less. In some embodiments, the ratio of marker-positive (e.g., c-kit positive) cells pre-depletion versus post-depletion is about 100:1 to about 3:1 (e.g., 50:1, 25:1, 15:1, 10:1, 5:1, and overlapping ranges therein). According to several embodiments, therapeutic populations of cells (e.g., post-depletion of c-kit and/or other markers) provide one or more of the following advantages: (i) reduced apoptosis of administered and/or endogenous cells, (ii) enhanced direct regeneration, (iii) reduction of competing (e.g., self-limiting) pathways upon administration, (iv) increased efficacy of the therapeutic cells (e.g., manifest as increase in the functionality and/or anatomic improvement of the diseased or damaged tissue), (v) a more uniform cell population leading to reduced adverse immune responses from a recipient, (vi) improved characterization of potency profile, (vii) reduced risk of teratoma formation, (viii) improved engraftment, (ix) more defined and uniform release of paracrine modulators, and (x) wider variety of administration routes, among other advantages.

In several embodiments, there is provided a composition of therapeutic cells for repairing or regenerating tissue, said composition comprising a therapeutic population of cells, wherein said therapeutic population of cells are obtained from non-embryonic donor tissue that is subjected to selective depletion, wherein said therapeutic population of cells is substantially depleted of cells expressing the c-kit stem cell marker, and wherein said therapeutic population of cells is suitable for repairing or regenerating tissue. In several embodiments, the donor tissue comprises cardiac tissue, and the therapeutic population of cells is depleted of at least about 90% of cells expressing the c-kit stem cell marker (as compared to the cells pre-depletion), wherein the therapeutic population of cells comprises cells of a size that is suitable for intracoronary delivery to the heart of a recipient, wherein the therapeutic population of cells retains the ability to engraft into the heart of the recipient, and wherein the therapeutic population of cells retains the ability of the cells to differentiate into cardiomyocytes. The therapeutic population of cells is further depleted of at least 90% of cells expressing the CD90 marker, wherein the therapeutic population of cells comprises cardiosphere-derived cells (CDCs), wherein the CDCs may be about 5 to about 35 microns in diameter and are suitable for systemic delivery to a recipient in need of repair of damaged or diseased cardiac tissue, and wherein the recipient is allogeneic with respect to the donor of the cardiac tissue.

In several embodiments, at least about 90% (e.g., about 91%, about 93%, about 95%, about 97%, about 99%, or ranges therein) of the therapeutic population of cells are positive for CD105. In several embodiments, the entire therapeutic population of cells is positive for CD105. In several embodiments, less than 10% (e.g., about 9%, about 7%, about 5%, about 3%, about 1%, or ranges therein) of the therapeutic population of cells are positive for CD45. In several embodiments, none of the cells in the therapeutic population are positive for CD45.

In several embodiments, there is provided a composition of therapeutic cells derived from donor tissue comprising a population of cells substantially depleted of cells expressing the c-kit stem cell marker. In several embodiments, there is provided a composition of therapeutic cells derived from donor cardiac tissue comprising a population of cells substantially depleted of cells expressing the c-kit stem cell marker. In several embodiments, the composition is depleted of at least about 90%, about 95% or about 99% cells expressing the c-kit stem cell marker (e.g., between about 90% to about 91%, about 91% to about 92%, about 92% to about 93%, about 94% to about 95%, about 95% to about 96%, about 96% to about 97%, about 97% to about 98%, about 98% to about 99%, and overlapping ranges thereof). For example, the therapeutic population of cells post-depletion versus pre-depletion comprises at least 90%, 95% or 99% fewer cells expressing the c-kit marker. In several embodiments, the composition is depleted of between about 99% and about 100% of the cells expressing the c-kit stem cell marker. In several embodiments, the composition is entirely depleted of cells expressing the c-kit stem cell marker. In several embodiments, the composition is depleted of cells that express other markers. The composition is depleted of at least 90%, about 95% or about 99% cells expressing CD90 (e.g., between about 90% to about 91%, about 91% to about 92%, about 92% to about 93%, about 94% to about 95%, about 95% to about 96%, about 96% to about 97%, about 97% to about 98%, about 98% to about 99%, and overlapping ranges thereof). For example, the therapeutic population of cells post-depletion versus pre-depletion comprises at least 90%, 95% or 99% fewer cells expressing CD90. In several embodiments, the composition is depleted of between about 99% and about 100% of the cells expressing CD90. In several embodiments, the composition is entirely depleted of cells expressing CD90. In several embodiments, however, lesser degrees of depletion still result in enhanced, improved, or undiminished therapeutic efficacy. In some embodiments, the composition is depleted of two or more subpopulations (e.g., depleted of cells expressing the c-kit stem cell marker and depleted of cells expressing CD90). In some embodiments, subpopulations bearing other markers (or combinations of markers) are depleted (in whole or in part) from the composition. In several embodiments, the depleted population is therapeutically effective based on its ability to produce, secrete, express, or otherwise generate a signal or signals, such as, for example, a paracrine signal (e.g., a cytokine, peptides, nucleic acid, microRNAs, lipids, exosomes or other species) that directly or indirectly promote tissue repair and/or regeneration by various mechanisms.

The population of cells (e.g., from which the subpopulation or subpopulations are depleted) comprises cardiosphere-derived cells (CDCs). In several such embodiments, the CDCs range from about 5 to about 35 microns in diameter. Advantageously, the size of CDCs in the composition renders the composition suitable for intracoronary delivery to a damaged heart, with a reduced risk of embolization at the arteriolar level. In several embodiments, the population of cells comprises cardiospheres (e.g., the depletion step has been performed on cardiosphere-forming cells, thereby resulting in depleted cardiospheres). In several embodiments, the population of cells comprises stem cells. In several embodiments, the composition further comprises cardiac cells. In some embodiments, the cardiac cells express at least one of the following cardiac markers: myosin heavy chain, myosin light chain, alpha sarcomeric actin, Troponin-T, nkx2.5, and GATA-4. Other markers of specific to (or associated with) a cardiac phenotype are expressed in some embodiments. In several embodiments, the composition further comprises endothelial cells. In several embodiments, the endothelial cells express at least one of the following endothelial markers CD105, KDR, flk-1, CD31, von Willebrand factor, Ve-cadherin, and smooth muscle alpha actin. Other markers specific of, or specific to (or associated with), an endothelial phenotype are expressed in some embodiments

Donor cardiac tissue can be obtained from a variety of sources, depending on the embodiment. In several embodiments, donor cardiac tissue is non-embryonic cardiac tissue. In several embodiments, donor cardiac tissue comprises tissue isolated from the crista terminalis of a donor heart. In several embodiments, donor cardiac tissue comprises tissue isolated from the right ventricular endocardium of a donor heart. In several embodiments, donor cardiac tissue comprises tissue isolated from the right ventricular septum of a donor heart. In several embodiments, donor cardiac tissue comprises tissue isolated from the septal or ventricular wall of a donor heart. In several embodiments, donor cardiac tissue comprises tissue isolated from the atrioventricular groove of a donor heart. In several embodiments, donor cardiac tissue comprises tissue isolated from the right and/or left atrial appendages of donor heart. In several embodiments, donor cardiac tissue comprises tissue collected from various regions of the heart and subsequently mixed. In several embodiments, donor cardiac tissue comprises tissue collected from an entire donor heart. In several embodiments, the entire heart may be obtained from an organ donor (e.g., a recently deceased donor).

Additionally, provided herein are methods for depleting the subpopulation (or subpopulations) of cells from the therapeutic population. In several embodiments, the composition is generated by exposing the population of cells to an anti-c-kit antibody that is coupled to a magnetic particle, and exposing the population of cells to a magnetic field, thereby specifically depleting the cells expressing the c-kit stem cell marker from the population of cells. Additionally, in several embodiments, the composition is generated by exposing the population of cells to a biotinylated anti-c-kit antibody, and exposing the population of cells to a surface comprising strepavidin/avidin, thereby specifically depleting the cells expressing the c-kit stem cell marker from the population of cells. In several embodiments, the composition is generated by exposing the population of cells to an anti-CD90 antibody that is coupled to a magnetic particle, and exposing the population of cells to a magnetic field, thereby specifically depleting the cells expressing the CD90 marker from the population of cells. Additionally, in several embodiments, the composition is generated by exposing the population of cells to a biotinylated anti-CD90 antibody, and exposing the population of cells to a surface comprising strepavidin/avidin, thereby specifically depleting the cells expressing the CD90 cell marker from the population of cells. In several embodiments, cells are exposed to both an anti-c-kit antibody that is coupled to a magnetic particle and an anti-CD90 antibody that is coupled to a magnetic particle and exposing the population of cells to a magnetic field, thereby depleting the population of both c-kit and CD90 subpopulations. The methods also, in several embodiments, can involve mixtures of magnetic and non-magnetic depletion. In several embodiments, the methods are repeated one or more times to increase the degree of depletion of the specific subpopulation to be removed. In several embodiments, multiple rounds of depletion can be performed to remove specific amounts of one or more certain subpopulations (e.g., depletion of a first subpopulation is substantially complete, while depletion of a second subpopulation is partial). Additional mechanisms of depletion are used in still additional embodiments (e.g., adherence of a subpopulation of cells to a solid surface and removal of the other cells, filtration to remove subpopulations of cells of a certain size, etc.).

In several embodiments, there are also provided methods of treating subjects having damaged or diseased cardiac tissue. In several embodiments, cells obtained from the subject are depleted of one or more subpopulations of cells, and then later reintroduced into that subject to regenerate the damaged or diseased tissue (e.g., an autologous transplant). In such embodiments, the method comprises obtaining plurality of cells harvested from the cardiac tissue of the subject, wherein the cells have been expanded in culture and harvested to yield a population of harvested cardiosphere-derived cells (CDCs), wherein the harvested CDCs have had one or more subpopulation of cells specifically depleted from the harvested CDC population, thereby generated depleted CDCs; and administering between about 1 x 10⁶ and about 100 x 10⁶ of the depleted CDCs to the subject, wherein the administered depleted CDCs generate one or more paracrine factors, wherein, after administration, at least a portion of the administered depleted CDCs engraft into the cardiac tissue of the subject; and wherein the one or more of the paracrine factors or the engraftment indirectly and/or directly improves the function and/or viability of the damaged cardiac tissue, thereby treating the subject. In several embodiments, the subject is an adult.

In several embodiments, there are provided methods of treating a first subject having damaged cardiac tissue with allogeneic cells from a second subject, the method comprising obtaining a plurality of cells harvested from the cardiac tissue of a second subject, wherein the cells have been expanded in culture and harvested to yield a population of harvested cardiosphere-derived cells (CDCs), wherein the harvested CDCs have had one or more subpopulation of cells specifically depleted from the harvested CDC population, thereby generated depleted CDCs; and administering between about 1 x 10⁶ and about 100 x 10⁶ of the depleted CDCs to the first subject, wherein the administered depleted CDCs generate one or more paracrine factors, wherein, after administration, at least a portion of the administered depleted CDCs engraft into the cardiac tissue of the first subject; and wherein the one or more of the paracrine factors or the engraftment indirectly and/or directly improves the function and/or viability of the damaged cardiac tissue, thereby treating the first subject. In several embodiments, the second subject is an adult, and hence the donor tissue is non-embryonic.

In several embodiments, the harvested CDCs are depleted of stem cells expressing the c-kit marker. In several embodiments, the harvested CDCs are depleted of cells expressing the CD90 marker. In several embodiments, the harvested CDCs are depleted of cells expressing both the c-kit and CD90 markers (and optionally cells expressing one or more additional markers). In several embodiments, the CDCs depleted may exhibit an enhanced ability to differentiate into cardiomyocytes and/or may promote a greater degree of direct regeneration than a non-depleted CDC population.

In several embodiments, there is also provided the use of a therapeutic composition comprising a population of cells depleted of cells expressing the c-kit stem cell marker for repair or regeneration of damaged or diseased cardiac tissue of a subject. In several embodiments, the use of the composition is for repairing cardiac tissue of a subject that is allogeneic with respect to the source of the donor cardiac tissue. In several embodiments, the use of the composition is for repairing cardiac tissue of the same subject (e.g., autologous).

In several embodiments, the composition is suitable for systemic administration. In several embodiments, the systemic administration comprises intravenous or intraarterial administration. In several embodiments, the composition is suitable for local administration. In several embodiments, the local administration comprises direct injection, perfusion, lavage or topical administration.

In several embodiments, administration of the composition results in a functional improvement in the damaged or diseased cardiac tissue. The functional improvement can be recognized by a variety of parameters, depending on the embodiment. For example, in several embodiments, administration of the composition results in increased viability of the damaged or diseased cardiac tissue. In several embodiments, this increased viability is associated an increased improvement in cardiac function (e.g., cardiac output). In several embodiments, the increased viability prevents a decline in heart function over time (as compared to the decline expected in the absence of the use of the composition).

The methods summarized above and set forth in further detail below describe certain actions taken by a practitioner; however, it should be understood that they can also include the instruction of those actions by another party. Thus, actions such as "administering a population of therapeutic cells depleted of cells expressing a marker" include "instructing the administration of a population of therapeutic cells depleted of cells expressing a marker."

### BRIEF DESCRIPTION OF THE DRAWINGS

Figures 1A-1B depict data related to efficacy of subpopulations of stem cells in cardiac stem cell therapy.
Figure 1C depicts a schematic of one embodiment of a method to generate subpopulations of cells.
Figures 2A-2B depict white-light microscopy analysis to confirm the depletion of c-kit^{POS} cells from a population of CDCs.
Figures 2C-2D depict confocal microscopy analysis to confirm the depletion of c-kit^{POS} cells from a population of CDCs.
Figures 2E-2F depict flow cytometry analysis to confirm the depletion of c-kit^{POS} cells from a population of CDCs.
Figures 2G-2J depict expression of different cell surface protein markers in populations of CDCs and c-kit^{DEP} CDCs.
Figure 3A depicts representative Masson's trichrome staining of cardiac tissue after treatment with various CDC populations, which relates to tissue viability.
Figures 3B-3E depict comparisons of various indicators of heart morphology protection and LV function augmentation between control, CDC- and c-kit^{DEP} CDC-treated hearts.
Figures 4A-4F depict confocal microscopy analysis of CDC and c-kit^{DEP} CDC on neonatal rat cardiomyocytes *in vitro.*
Figures 4G-4I depict comparisons of cardiomyocyte growth and function in cells treated with CDC and c-kit^{DEP} CDC.
Figures 5A-5C depict endothelial cell tube formation on Matrigel in control, CDC- or c-kit^{DEP} CDC-treated cardiomyocytes.
Figure 5D depicts HUVEC tube formation as a function of tube length in CDC- and c-kit^{DEP} CDC-treated cardiomyocytes.
Figures 6A-6F depict comparisons of expression levels of different paracrine factors between CDC- and c-kit^{DEP} CDC-treated cells.
Figures 7A-7C depict engraftment of control, CDCs and c-kit^{DEP} CDCs in post-MI mouse hearts.
Figures 7D-7E depict differentiation of CDC and c-kit^{DEP} CDCs into cardiomyocytes in post-MI mouse hearts.
Figures 7F-7G depict comparisons of CDC and c-kit^{DEP} CDC before and after engraftment into post-MI mouse hearts.
Figures 8A-8D depict differentiation of CDC and c-kit^{DEP} CDC into smooth muscle and endothelial cells after transplantation into post-MI hearts.
Figures 9A-9C depict cardiomyocyte cycling promoted by CDCs and c-kit^{DEP} CDCs.
Figure 9D depicts a comparison of cardiomyocyte cycling in control, CDCs and c-kit^{DEP} CDCs.
Figures 10A-10C depict promotion of angiogenesis in CDC- and c-kitDEP CDC-treated cells compared to control.
Figure 10D depicts a comparison of vWF^{POS} vessels in control, CDC- and c-kit^{DEP} CDC-treated cells.
Figure 11 depicts a negative correlation between CD90 expression in CDCs used to treat infarction and scar percentage at 6 months. Thus, CDCs with less CD90-positive cells showed higher scar reduction potency.
Figure 12 depicts a negative correlation between CD90 expression in CDCs used to treat infarction and scar percentage at 12 months. Thus, CDCs with less CD90-positive cells showed higher scar reduction potency.
Figures 13A-13D depict expression of certain markers on CDCs that were prepared for use in a clinical trial. Figure 13A shows the percentage of CDCs expressing the marker CD105 in each of 17 preparations, as well as the average. Figure 13B shows the percentage of CDCs expressing the marker CD45 in each of 17 preparations, as well as the average. Figure 13C shows the percentage of CDCs expressing the marker c-kit in each of 17 preparations, as well as the average. Figure 13B shows the percentage of CDCs expressing the marker CD90 in each of 17 preparations, as well as the average.
Figure 14 depicts a correlation between those CDC populations expressing high levels of CD90 or low levels of CD90 with the change in scar formation that was realized during a clinical trial.
Figures 15A-15B are graphical depiction of the phenotypic composition of CDC's based on their marker expression (A) and a breakdown of various populations of CDCs that were experimentally tested against one another, as discussed in more detail below.
Figures 16A-16B depict an experimental protocol used, in several embodiments, to deplete certain subpopulations from the CDC population. Figure 16A depicts a schematic protocol for magnetically separating out certain subpopulations. Figure 16B depicts one embodiment of an experimental system used to accomplish depletion of subpopulations.

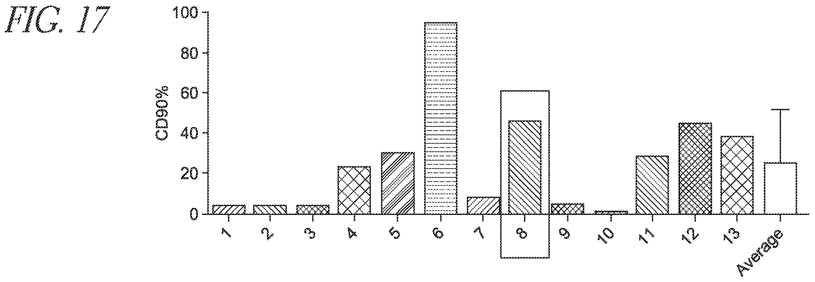

Figure 17 identifies one specific CDC preparation that was selected for certain depletion studies. CDC preparation 8 (shown with a box around it) was selected based on the preparation having roughly 40% CD90+ CDCs.
Figures 18A-18D depict experimental data regarding the specificity a lack of toxicity of depletion of CD90 subpopulations. Figure 18A depicts flow cytometry based analysis of the CDC population. Figure 18C shows the growth of the undepleted CDCs in culture. Figure 18B depicts flow cytometry analysis of the CDCs after depletion of the CD90+ subpopulation. Figure 18D shows that CDCs grow with normal morphology even after depletion of the CD90+ subpopulation.
Figures 19A-19B depict fluorescent immunohistochemical analysis of the expression of CD90 and CD105 by CDCs. Figure 19A depicts cultured CDCs without depletion and expression of CD105 (throughout the cells), CD90 arrows, and cell nuclei (arrowheads). Figure 19B depicts the same analysis of the CDCs after depletion of the CD90+ subpopulation. The depleted CDCs retained CD105 expression and nuclei, but CD90 was not detected.
Figures 20A and 20B depict functional benefits of various CDC populations, namely those having been depleted of the ckit⁺ subpopulation, those depleted of the CD90⁺ subpopulation, and those depleted of both ckit⁺ and CD90⁺ subpopulations. Figure 20A depicts the left ventricular ejection fraction (LVEF) at baseline for while Figure 20B depicts LVEF for each respective CDC population at three weeks post-administration.
Figures 21A-21B depict the correlation between CD90 expression and therapeutic benefits according to some embodiments, as measured by the change in ejection fraction. Figure 21A depicts a negative correlation between CD90⁺ expression in the change in left ventricular ejection fraction. Figure 21B depicts the statistical trendline resulting from this data.

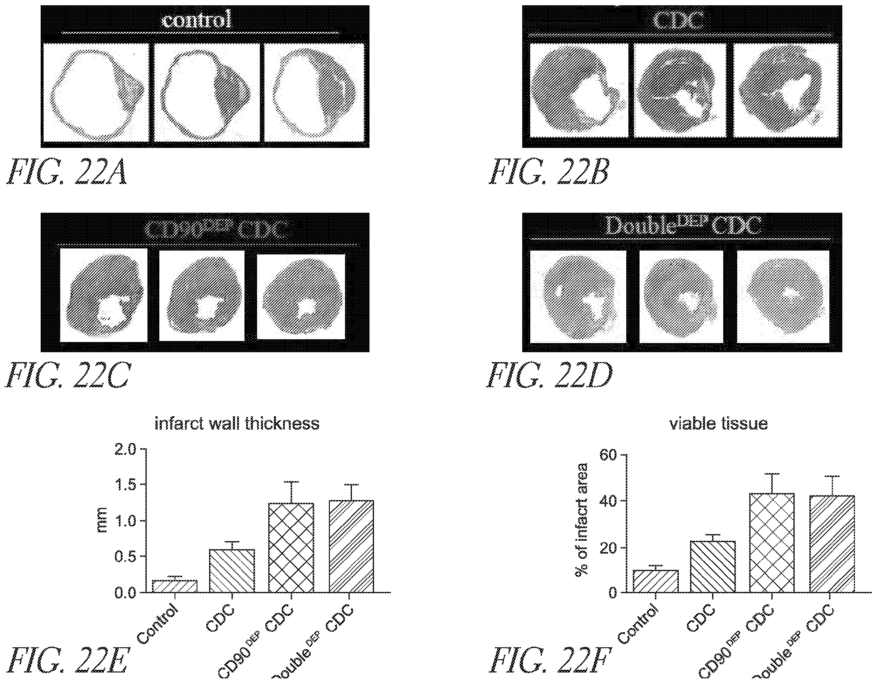

Figures 22A-22F depict various morphometric measurements of cardiac tissue post-myocardial infarction, and post-administration of one of the various treatments according to several embodiments disclosed herein. Figure 22A depicts control tissue post infarction. Figure 22B depicts post-MI tissue treated with non-depleted CDCs. Figure 22C depicts morphometric measurements of post-MI tissue after administration of CD90⁺ depleted CDCs. Figure 22D depicts post-MI tissue after the delivery of ckit⁺ and CD90⁺ depleted CDCs. Figure 22E depicts summary data of infarct wall thickness for each of the various CDC populations in Figures 22A-22D, and Figure 22F depicts the change in viable tissue after treatment with each of the various CDC populations.

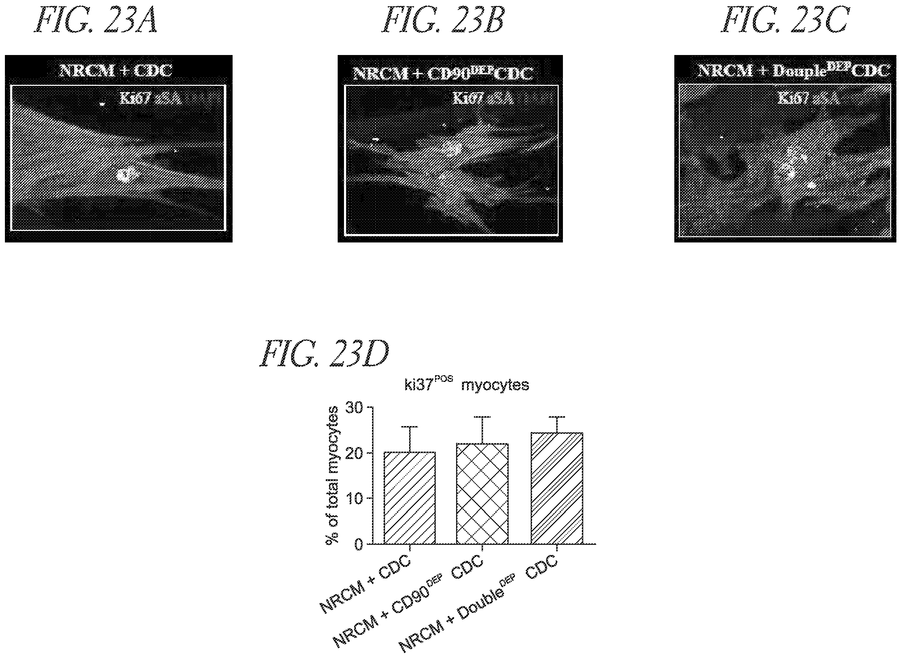

Figures 23A-23D depict the results of cardiomyocyte proliferation assays for various non-depleted and depleted CDC populations disclosed herein. Figure 23A shows the results of proliferation of neonatal rat cardiomyocytes cultured with CDCs that have not been depleted of any subpopulation. Figure 23B depicts the co-culture of neonatal rat cardiomyocytes with CDC population that has been depleted of cells that are CD90⁺. Figure 23C depicts the results of co-culture of neonatal rat cardiomyocytes with the CDC population that has been doubly depleted of both CD90⁺ cells and ckit⁺ cells. Figure 23D provides a summary of these results, depicting the percentage of proliferating myocytes (as measured by Ki67 positivity) for each of the groups described above.
Figures 24A-24D depict the result of cardiomyocyte apoptosis assays. Figure 24A depicts neonatal rat cardiomyocytes co-cultured with CDCs, Figure 24B depicts neonatal rat cardiomyocytes co-cultured with CDCs that have been depleted of the CD90⁺ subpopulation, Figure 24C depicts the co-culture of neonatal rat cardiomyocytes with CDCs that have been depleted of both CD 90 positive cells and seek it positive cells. Figure 24D summarizes these results with respect to the frequency of the TUNEL positive myocytes, which is indicative of apoptosis.
Figures 25A-25D depict the results of cytokine array analysis. Each of the boxes depicted are positioned in the corresponding related figure, in other words a in the same position in Figure 25A relates to the same cytokine as the box in the corresponding position in Figure 25B, C, or D. Figures 25A depicts the change in expression of bFGF, EGF and VEGF in CDC populations and CD 90⁺-depleted CDC populations.
Figures 26A-26B depict summary data related to the expression of various growth factors by either CDC populations or CD 90⁺-depleted CDC populations. Figure 26A shows summary data of the intensity of the cytokine assay results from Figure 25. Figure 26B depicts specific analysis of VEGF release from CDCs, on the left, as compared to CD90⁺-depleted CDC populations, on the right.
Figures 27A-27D depict additional cytokine expression analysis of GM-CSF, MCP3, RANTES, IL-1 alpha, and IL-1 beta from CDCs or CDCs depleted of CD90⁺ cells.
Figure 28 is a histogram depicting the relative release GM-CSF, MCP3, RANTES, IL-1 alpha, and IL-1 beta from CDCs or CDCs depleted of CD90⁺ cells.
Figure 29 depicts the fluorescent immunohistochemistry of doubly depleted CDCs (e.g., CDCs depleted of both ckit⁺ cells and CD90⁺ cells). The Figure also depicts that, in several embodiments, CDCs are positive for CD105 and/or alpha sarcomeric actin.

### DETAILED DESCRIPTION

Many diseases or injuries surpass the natural regenerative capacity of an organism. Cell therapy, which involves the administration of cells to tissue (or multiple tissues) to treat a disease or injury, is rapidly gaining ground as a first line medical treatment method. In several embodiments described herein, methods of obtaining, processing, and administering therapeutic cells to a recipient are provided. Several such embodiments are used in the treatment of damage resulting from a cardiac disease, which can result in (but is not limited to) improvement in cardiac function and/or regeneration of cardiac tissue in the recipient. In several embodiments, the cardiac disease is the result of one or more of an acute heart failure and/or chronic heart failure. In some embodiments, the disease creates damage to the cardiac tissue due to one or more of ischemia, reperfusion, or infarction. In alternative embodiments, other tissues are treated using the methods and/or therapeutic cell compositions disclosed herein (including, but not limited to, for example, liver, nervous tissue, kidney, lung, gastrointestinal tract, etc.)

### General

As used herein, the term "therapeutic cells" shall be given its ordinary meaning and shall include stem cells, progenitor cells, as well as cells that have the potential to regenerate tissue (or initiate a regenerative process) over the lifetime of that cell. Therapeutic cells, in several embodiments, comprise mixed cell populations. Depending on the embodiment, the mixed cell populations may be a result of the process of obtaining the cells (e.g., results from the expansion of an initial cell or tissue source), while in some embodiments, the mixture of cells results from a tailored combination of various cell types that have already been obtained. As one non-limiting example, a mixed population that may be used in the context of cardiac tissue repair or regeneration is the cardiosphere. In several embodiments, the populations comprise separate cell types, primarily pure cell populations, substantially pure populations, and/or pure populations. Derivative cell types are also used in several embodiments, such as for example, the cardiosphere-derived cell (CDC) or a subsequently produced generation of cardiospheres (IICSps). Therapeutic cells include cells that directly repair tissue (e.g., due to the cells themselves) and cells that promote tissue repair indirectly (e.g., via release of paracrine factors, or induction of other pro-regenerative signals or events).

The source of the therapeutic cells varies, depending on the embodiment. In several embodiments, the source of the population of therapeutic cells is from a subject who will eventually receive the cells (e.g., autologous cell therapy). In some embodiments, autologous cell therapy is advantageous due to the significant reduction in possible adverse immune effects upon administration of the therapeutic cells. However, autologous cell therapy, in certain instances, involves a delay between the generation of the therapeutic cells (e.g., harvesting the cells from a donor tissue) and subsequent administration of the therapeutic cells, the delay being a result of the time needed for expansion of the isolated therapeutic cells to a population of sufficient cell number to provide efficacious therapy. In several embodiments, however, the donor of the tissue from which the therapeutic cells are derived is distinct from the subject will eventually receive the therapeutic cell population (e.g., allogeneic cell therapy). Allogeneic cell therapy thus provides particular advantages, in some embodiments, because a subject can receive a population of therapeutic cells in an "off-the-shelf' manner. This approach, in several embodiment, reduces and/or minimizes the delay between an event that damages tissue (e.g., an acute injury or event such as a heart attack), and the administration of therapeutic cells. In several embodiments, a dose of therapeutic cells can be taken from a pre-existing cell bank. Depending on the embodiment, the subject receiving the therapeutic cells may be type matched to the donor cells, thereby reducing immune rejection issues. However, in several embodiments, allogeneic cell therapy does not present immune issues that significantly compromise the beneficial effects (e.g., even with some immune response against the administered cells, therapeutic efficacy is achieved). In several embodiments, allogeneic cell therapy broadens scope of possible donor tissue. For example, cadaver tissue, once cleared as being of sufficient quality, can be used to generate multiple populations of therapeutic cells. Advantageously, this allows the generation of a substantial number of therapeutic doses for immediate therapeutic use, or alternatively for storage for future therapeutic use. In several embodiments, xenogeneic cell therapy methods and compositions are provided, while in still additional embodiments, syngeneic cell therapy methods and compositions are provided. Regardless of the type of therapeutic cell (e.g., autologous or allogeneic), the methods disclosed herein allow for substantial expansion of the cell population, which enables use of a relatively small amount of starting material (e.g., donor tissue). Thus, using the methods disclosed, the larger the amount of suitable donor tissue, the greater number of therapeutic doses can be generated.

Depending on the embodiment, a variety of different cells may be used for cell therapy. As used herein, the term "stem cell" shall be given its ordinary meaning and shall also be referred to as a cell that has the capacity to self renew (e.g., mitotically divide) while maintaining an undifferentiated state and has the capacity to differentiate into multiple cell types. As used herein, the term "progenitor cell" shall be given its ordinary meaning, and shall also refer to a cell that, like a stem cell can differentiate into multiple cell types, but is already more differentiated down a particular developmental pathway.

Depending on the tissue to be treated and the therapeutic goals, a therapeutic cell population may be chosen, at least in part, based on its potency. For example, in some embodiments, totipotent stem cells are used, these cells being able to differentiate into both embryonic and extraembryonic cell types, thereby enabling the generation of a complete organism. In some embodiments pluripotent cells are used. Pluripotent cells are derived from totipotent cells, and can differentiate into nearly all cell types, including those derived from endoderm, mesoderm, and/or ectoderm. In still additional embodiments, multipotent cells are used. Multipotent cells can differentiate into a number of different cell types, however, the resultant cells are typically of a relatively closely related family. Oligopotent stem cells are used in certain embodiments, the cells being able to differentiate into a limited number of cell types. Finally, in some embodiments, unipotent stem cells are used, the cells being able only to self renew and not differentiate into any other type of cell. Although in several embodiments, the therapeutic cells are stem cells, in several embodiments, the therapeutic cells need not be stem cells. For example, the therapeutic cells may secrete beneficial paracrine factors (cytokines, peptides, microRNAs, lipids or other species) that promote tissue repair and/or regeneration by various mechanisms, independent of the sternness of the therapeutic cells.

In several embodiments, adult stem cells are obtained and used in cell therapy. For example, in the context of treating cardiac disease or damage, cardiospheres and/or CDCs make up at least a portion of the therapeutic cell population. Populations of CDCs and/or cardiospheres comprise, depending on the embodiments, stem cells, cardiac cells, and/or endothelial cells. In some embodiments, populations of CDCs and/or cardiospheres may comprise other undefined cell types. Thus, these cell populations, when used in cardiac cell therapy, have the potential to regenerate not only the cardiac musculature, but also the cardiac vasculature and smooth muscle that is needed to supply blood and oxygen to that muscle tissue. In additional embodiments, induced pluripotent stem cells are generated and used. In still additional embodiments, mixtures of cell types may be used.

### Methods of Harvesting Tissue and Production of Therapeutic Cells

As discussed above, it shall be appreciated that the term "therapeutic cells" as used herein refers not only to mixed cell populations described herein (e.g., cardiospheres, CDCs, and/or exogenously mixed populations) but also to the cell populations which have been selectively depleted (partially or completely) of one or more subpopulation of cells (e.g., based on their expression of one or more markers or other features). In some embodiments, the composition that is depleted (partially or fully) of one or more subpopulations of cells may be termed "pure" or "purified". As used herein, the term "removed subpopulation" shall be given its ordinary meaning, and shall also refer to those cells specifically identified and removed from the complete population of therapeutic cells resulting from the processing of donor tissue. As used herein, the term "depleted population" shall be given its ordinary meaning and shall also refer to the population of therapeutic cells remaining after the selective removal of one or more subpopulations

Donor tissue may be obtained from adult sources, though adults non-embryonic) tissue sources are preferred for several embodiments. In some embodiments, donor cardiac tissue is obtained during a surgical procedure (such as a cardiac bypass surgery in which a surgeon would already have ready access to the cardiac tissue). In some embodiments, specific tissue procurement methods/procedures are employed. For example, biopsies may be performed to obtain small pieces of donor tissue. As discussed above, depending on the embodiment, the donor tissue may be allogeneic, autologous (or xenogeneic or syngeneic) with respect to the ultimate recipient of the therapeutic cells. In several embodiments, percutaneous biopsies (e.g., percutaneous endomyocardial biopsy) are performed to obtain donor tissue. In other embodiments, larger quantities of tissue are obtained from recently deceased organ donors (including cadavers in suitable condition for organ donation). The methods disclosed herein are particularly advantageous in that they result in significant expansion of cell numbers. To that end, a small piece (or pieces) of starting tissue can result, depending on the embodiment, in an expanded number of therapeutic doses. In several embodiments, the starting material can comprise tissue samples of about 1 mg to about 50 mg. In some embodiments of cardiac stem cell generation, the mass of a cardiac tissue sample obtained by biopsy can be about 25 mg or less. A biopsy sample of this size can result in approximately 10 to 25 million cells being available for implantation into the coronary arteries (of the same subject or an allogeneic subject) in about 3 to about 6 weeks (e.g., about 4 weeks). When allogeneic cell therapy is to be used, it shall be appreciated that the cell processing can occur in advance of an administration of the therapeutic cells, the timing of which can reduce or substantially eliminate delay in therapeutic administration (e.g., the cells are "ready to use"). For cardiac cell therapy, tissue sample may be obtained from a variety of locations in the heart, including but not limited to, the crista terminalis, the right ventricular endocardium, the right ventricular septum, the septal or ventricle wall, the atrioventricular groove, the right and/or left atrial appendages, or an entire heart (in the context of organ donation). When other organs are used as the source of therapeutic cells, alternative methods may be used to access those organs, which may also include biopsy, surgical means, or combinations thereof. It shall be appreciated that, as a result of the expansion in population size from the disclosed methods, the larger the amount of starting material, the larger potential number of therapeutic doses can be generated.

As a non-limiting example (e.g., the basic procedures can be applied to other tissue sources), disclosed below is one embodiment of cardiac tissue processing to generate populations of cardiac stem cells. Cardiac tissue samples (e.g., from biopsy or a donor heart) are weighed, dissected into small fragments free of connective tissue. The fragments are optionally washed (e.g., in phosphate-buffered saline) to remove lysed cells (e.g., red blood cells, or dissociated non-viable heart cells). Optionally, the small tissue fragments are partially digested with a protease. Non-limiting examples of suitable proteases include collagenase, trypsin, chymotrypsin, pepsin, thrombin, plasmin, elastase, cathepsin, etc. Post-digestion, the partially digested fragments are cultured (as tissue "explants"). Depending on the tissue source and the relative ease by which cells are liberated from the tissue, the digested pieces of tissue range in size from about 0.1 mm² to about 2.5 mm². In several embodiments, the digested pieces of tissue range 0.25 mm² to about 1.5 mm². Smaller or larger pieces of tissue can be used in other embodiments. In several embodiments, the size of the tissue fragment is selected based on the cellular density of the tissue, in order to ensure that nutrients and oxygen can permeate the tissue sufficiently to maintain the viability of the inner-most portions of the tissue.

In several embodiments, the culture surface and/or culture media are selected or treated to encourage the tissue fragments to adhere (at least partially) to the culture surface. In some embodiments, the culture surface is supplemented with fibronectin or other extracellular matrix (ECM) proteins, such as collagen, elastin, gelatin and laminin, for example. In other embodiments, the culture surface is treated with plasma. In certain embodiments, the dishes are coated with fibronectin at a final concentration of from about 10 to about 50 µg/mL. In still other embodiments, the fibronectin dishes are coated with fibronectin at a final concentration of from about 20 to 40 µg/mL, with still other embodiments employing a final fibronectin concentration of about 25 µg/mL. Greater or lesser concentrations are used in other embodiments, depending on the size of the tissue fragments. In some embodiments, the culture surface is manually disrupted (e.g., scraped) to provide a 3-dimensional surface that encourages adherence of the tissue fragments to the surface. In several embodiments, the culture surface is untreated.

In certain embodiments, the base component of the complete explant medium comprises Iscove's Modified Dulbecco's Medium (IMDM). Other cell media may be used in other embodiments. In some embodiments, the culture media is supplemented with fetal calf serum (FCS) or fetal bovine serum (FBS). In certain embodiments, the media is supplemented with serum ranging from about 5% to about 30% v/v, including about 5% to about 10% v/v, about 10% to about 15% v/v, about 15% to about 20% v/v, about 20% to about 25% v/v, about 25% to about 30% v/v, and overlapping ranges thereof. In other embodiments, the culture media is serum-free. In some serum-free embodiments the media is optionally supplemented with specific growth factors and/or hydrolyzed plant extracts. In other embodiments, the media is supplemented with serum, but no additional exogenous growth factors. In yet other embodiments, the media is further supplemented with antibiotics, essential amino acids, reducing agents, or combinations thereof. In one embodiment, the explant medium comprises IMDM supplemented with about 20% fetal bovine serum, about 50 µg/mL gentamicin, about 2 mM L-glutamine, and about 0.1mM 2-mercaptoethanol. In some embodiments, the explant media is changed every 2-4 days while the explants are cultured, however it shall be appreciated that alternative medias and times that are functionally substantially similar to those disclosed herein may also be used.

The adherent explants are cultured until a layer of stromal-like cells arise from the explants. Subsequently, small, round, phase-bright cells that appear and migrate over the stromal-cells. In certain embodiments, the explants are cultured until the stromal-like cells grow to confluence. At or before that stage, the phase-bright cells are harvested. In certain embodiments, phase-bright cells are harvested by manual methods, while in others, enzymatic digestion, for example trypsin, is used. In several embodiments, or TryPEL Select™ is used for enzymatic digestion. The phase-bright cells may be termed cardiosphere-forming cells, and the two phrases are used interchangeably herein.

Cardiosphere-forming cells are then cultured in cardiosphere media. In certain embodiments, the culture surface is coated with, for example, poly-D-lysine, or another suitable natural or synthetic molecule to deter cell attachment to the dish surface. In other embodiments, for example, laminin, fibronectin, poly-L-ornithine, or combinations thereof may be used. In several embodiments, the culture surface is untreated.

In certain embodiments, the base component of the cardiosphere medium comprises Iscove's Modified Dulbecco's Medium (IMDM). Other cell media may be used in other embodiments. In some embodiments, the culture media is supplemented with fetal calf serum (FCS) or fetal bovine serum (FBS). In certain embodiments, the media is supplemented with serum ranging from about 5% to about 30% v/v, including about 5% to about 10% v/v, about 10% to about 15% v/v, about 15% to about 20% v/v, about 20% to about 25% v/v, about 25% to about 30% v/v, and overlapping ranges thereof. In other embodiments, the culture media is serum-free. In some serum-free embodiments the media is optionally supplemented with specific growth factors and/or hydrolyzed plant extracts. In other embodiments, the media is supplemented with serum, but no additional exogenous growth factors. In yet other embodiments, the media is further supplemented with antibiotics, essential amino acids, reducing agents, or combinations thereof. In one embodiment, the cardiosphere medium comprises IMDM supplemented with about 20% fetal bovine serum, about 50 µg/mL gentamicin, about 2 mM L-glutamine, and about 0.1mM2-mercaptoethanol. In some embodiments, the explant media is changed every 2-4 days while the explants are cultured, however it shall be appreciated that alternative medias and times that are functionally substantially similar to those disclosed herein may also be used.

In several embodiments, spontaneous formation of cardiospheres results from the culturing of the cardiosphere forming cells. Cardiospheres are spherical multicellular clusters in the culture medium. In several embodiments, cells that remain adherent to the culture dishes are discarded. As discussed below, in several embodiments, the cardiospheres are collected, processed (e.g., depleted of one or more subpopulation of cells) and used in cell therapy to repair or regenerate cardiac tissue. In other embodiments, they are processed and seeded into a biomaterial or synthetic graft. In other embodiments, the cardiospheres are further cultured on coated cell culture flasks in cardiosphere-derived stem cell (CDC) medium.

CDCs result, in several embodiments, from the culturing of cardiospheres under conditions that promote the attachment and subsequent monolayer expansion of the cardiospheres. In several embodiments, the culturing vessels are optionally fibronectin coated, though in other embodiments other cellular attachment promoting coatings are employed. In several embodiments, the medium for CDC growth comprises IMDM, optionally supplemented with fetal calf serum (FCS) or fetal bovine serum (FBS). In some embodiments, the media is supplemented with serum ranging from 5 to 30% v/v, including about 5% to about 10% v/v, about 10% to about 15% v/v, about 15% to about 20% v/v, about 20% to about 25% v/v, about 25% to about 30% v/v, and overlapping ranges thereof. In other embodiments, the culture media is serum-free and may optionally be supplemented with specific growth factors and/or hydrolyzed plant extracts. In certain other embodiments, the media is further supplemented with antibiotics, essential amino acids, reducing agents, or combinations thereof. In one embodiment, the CDC medium comprises IMDM supplemented with about 10% fetal bovine serum, about 2 mM L-glutamine, and about 0.1mM 2-mercaptoethanol.

In several embodiments, CDCs are repeatedly passaged (e.g., removed from the culture surface, washed to remove dead cells, divided into multiple aliquots and replated). In some embodiments, rather than passaging to generate additional CDCs, the harvested CDCs and plated on poly-D-lysine-coated dishes to form a second generation of cardiospheres (IICSps). This process of generating cardiospheres followed by CDCs followed by a subsequent generation of cardiospheres may be repeated as needed to expand the population of any of the cardiac-derived therapeutic cell types.

In several embodiments, the processing of a cardiac sample yields therapeutic cells that comprise a mixed population of cells (for example, stem cells, cardiac cells, and /or vascular cells, among other cell types). In some embodiments, each of these types of cells comprises one or more subpopulations. In some embodiments, the various cell types express one or more identifying markers. As discussed in greater detail below, in several embodiments, one or more subpopulation of cells is removed based, at least in part, one expression of one or more markers.

### Methods of Removing Subpopulations of Therapeutic Cells

In several embodiments, the mixed population of therapeutic cells comprises a number of distinct cell types, as discussed herein. In some embodiments, the various types of cells comprise a multiple subpopulations of cells. In some embodiments, these subpopulations can be distinguished from one another, at least in part, based on their expression of certain markers (e.g., stem cell or endothelial cell markers). In some embodiments, the expression of a single marker is enough to identify a subpopulation of cells (e.g., the marker is unique to that subpopulation). In some embodiments, it is the combination of a plurality of markers that are expressed by cell subpopulation that allow for identification of that subpopulation.

Depending on the embodiment, one or more of a variety of methods to selectively remove (e.g., deplete) one or more subpopulations of therapeutic cells may be used. After removal, these subpopulations may be discarded, and the remaining population of therapeutic cells can be used in cell therapy.

In several embodiments, an antibody directed against a marker, known to be present on the subpopulation of cells to be removed is used to capture this subpopulation of cells. Subsequently, this antibody (now bound to the cells to be removed) can be recognized, in one of a variety of ways. In several embodiments, the antibody is coupled to one more magnetic particles. As such, passage of the antibody-subpopulation complex through a magnetic field results in the selective retention of the subpopulation of cells to be removed. In other words, the desired population of therapeutic cells, now depleted of this specific subpopulation, freely passes through the magnetic field and can be collected for subsequent use. In some embodiments, two or more passages through the magnetic field are used to increase the overall removal of the subpopulation.

In other embodiments, nonmagnetic recognition of the antibody-subpopulation complex is used to selectively remove subpopulation of cells. For example, antibodies, bearing a biotinylation moiety can be selectively removed from a cell mixture based on an interaction between the biotinylation moiety and avidin/streptavidin beads. Similar to the magnetic approach, this method results in the selective removal of a desired subpopulation, leaving a population of therapeutic cells that have been filtered of that specific subpopulation.

Selective filtration of a cell mixture can also be achieved, in some embodiments, through the use of contactless dielectrophoresis. In such a method, unique electrical signatures of desired versus undesired cells can be recognized and used to selectively remove the undesired cells (e.g., the subpopulation to be removed).

In still additional embodiments, size exclusion can be used to selectively remove undesired subpopulations of cells. For example, simple filtration could be used to remove (e.g., by retention on a filter) a cell subpopulation that are larger than the desired therapeutic cells. Conversely, should the therapeutic cells in a particular embodiment be larger in size the cells to be removed, the therapeutic cells could be trapped on a filter, while the undesired cells are allowed to pass through. The trapped therapeutic cells could then be removed from the filter and paired for subsequent use or storage. Similarly, size exclusion chromatography is used, in certain embodiments, to selectively distinguish between subpopulations of cells to be removed and the desired therapeutic cells. Additionally, highly specific exogenous labels could be used to tag one or more undesired subpopulations for subsequent removal from the desired therapeutic cells. Flow cytometry is also used for selective filtering (or quality control analysis of depleted cell populations) of desired and undesired populations, in certain embodiments. Additionally, immunohistochemical identification and select isolation (e.g., removal and retention of desired cells or removal and subsequent discarding of undesired cells) can be used to generate population of therapeutic cells that has been filtered of undesired cells.

As used herein, the terms "filtered", "screened", "depleted", "removed", "gated", and "partitioned" shall be given their ordinary meaning, and shall also refer to the removal (either partial, complete, or substantially complete) of a particular type of cell subpopulation from a larger population of cells. Depending on the embodiment, those cells that are filtered or removed, etc., may be the desired therapeutic cells or alternatively may be an undesired (or simply unnecessary) subpopulation of cells.

In some embodiments, multiple rounds of subpopulation removal are performed. In some embodiments, this is to ensure an increased overall removal of a particular subpopulation. In some such embodiments, a first-round and subsequent rounds are directed against the same marker expressed by subpopulation of cells to be removed. In some embodiments, the subpopulation of cells to be removed, expresses a plurality of markers and therefore one or more rounds may be performed directed against a first marker, and one or more rounds may be performed against a second marker. It should be appreciated that additional rounds may also be performed as necessary (either against the first or second marker or against additional markers).

In some embodiments, more than one subpopulation of cells is removed, either by using a plurality of removal methods, the same removal method directed against different cell subpopulations, or combinations thereof.

As discussed above, the methods disclosed herein for treating a donor tissue and obtaining a resultant population of therapeutic cells results in a plurality of cells expressing a variety of different markers. Some cells express stem cell markers, while other cells express markers more specific to their cell type, e.g. markers of differentiated cells. Certain cells express combinations of stem cell markers and markers of differentiated cells, depending on the status of that particular cell. The individual markers, or combination of markers, may be used in various embodiments to selectively remove (or retain) a particular subpopulation of cells.

For example, subpopulations of stem cells bearing one or more of the following markers CD105, CD90, CD34, Sca-1, and c-kit can be selectively removed from the larger population of therapeutic cells.

Vascular or endothelial cells bearing one or more of the following markers Flk-1, smooth muscle cell specific myosin heavy chain, vascular endothelial cell cadherin, CD34 can be selectively removed from the larger population of therapeutic cells.

Bone-derived cells bearing one or more of the following markers bone specific alkaline phosphatase, hydroxyapatite, osteocalcin can be selectively removed from the larger population of therapeutic cells.

Bone-marrow-derived cells bearing one or more of the following markers bone that morphogenetic protein receptor, CD4,CD8, CD34, CD 34+/sca1+/lin-, CD38, CD44, colony forming unit (CFU), fibroblastic colony forming unit (FCFU), CD45 , Mac-1, lineage surface antigen, Muc 18, (also known as CD146), Sca1, Stro-1 antigen, and Thy-1 can be selectively removed from the larger population of therapeutic cells.

Cartilage derived cells bearing one or more of the following markers collagen type II or type IV, keratin, or sulfated proteoglycan, can be selectively removed from the larger population of therapeutic cells.

Adipose-derived cells bearing one or more of the adipocyte lipid binding protein and/or fatty acid transporter markers can be selectively removed from the larger population of therapeutic cells.

Nervous tissue cells bearing one or more of the following markers CD 133, glial fibrillary acidic protein (GFAP), microtubule associated protein-2, myelin basic protein, nestin, neural tubulin, neurofilament, neurosphere, noggin, O4, O1, synaptophysin, and tau can be selectively removed from the larger population of therapeutic cells.

Pancreatic cells bearing one or more of the following markers cytokeratin 19, glucagon, insulin, insulin promoting factor I, nestin, pancreatic polypeptide, and somatostatin can be selectively removed from the larger population of therapeutic cells.

Pluripotent stem cells (e.g., iPS cells) bearing one or more of the following markers alkaline phosphatase, alpha-fetoprotein, bone morphogenetic protein 4, brachyury, CD30, crypto, GATA-4, GCTM-2, Genesis, germ cell nuclear factor 4, nestin, neuronal cell adhesion molecule, Oct4, Pax6, stage specific embryonic antigen three, stage specific embryonic antigen four, telomerase, TRA-1-60, TRA-1-81, and vimentin can be selectively removed from the larger population of therapeutic cells.

Muscle cells (e.g., skeletal muscle, cardiac, smooth muscle) bearing one or more of the following markers MyoD, Pax7, Myogenin (MR4), myosin heavy chain, myosin light chain, smooth muscle alpha actin, alpha sarcomeric actin can be selectively removed from the larger population of therapeutic cells.

It shall be appreciated that the listing of any one marker in a single category above does not necessarily mean that that marker belongs only to that category. Rather, it shall be appreciated that there may be overlap amongst the markers in the various categories (e.g., a single cell may express markers from more than a single category of marker. It shall also be appreciated, that while in several embodiments, the use of cell surface markers are preferred for identification and specific removal of subpopulations of cells, other aspects of the cell's expression profile (e.g., DNA, mRNA, protein) may be used to selectively identify and remove a subpopulation of cells.

Moreover, in several embodiments, it is the combination of the expression of a certain marker (or markers) coupled with the lack of expression of a certain marker (or markers), that allow for the specific removal of a subpopulation of cells.

In several embodiments, the specific removal of one or more subpopulations results in one or more therapeutic advantages. In several embodiments, the removal of one or more subpopulations of cells results in increased efficacy of the depleted therapeutic cells. In several embodiments, the increased efficacy is manifest as an increase in the functionality of the diseased or damaged tissue. As a non-limiting example, administration of a population of therapeutic cells that has been depleted (partially or completely) of certain subpopulations of stem cells (e.g., c-kit positive cells) results in increased cardiac function post-therapy, as compared to cardiac function post-injury. In several embodiments, the increase in function comprises an increase in at least one of left ventricular percent fractional area and left ventricular ejection fraction. In several embodiments, increases of at least about 5%, 10%, 15%, or more are realized. In some embodiments, ejection fraction is increased by about 5%. In some embodiments, ejection fraction is increased by about 10%. In some embodiments, ejection fraction is increased by about 15%. In some embodiments, ejection fraction is increased by about 20%. In some embodiments, ejection fraction is increased by up to about 25%. In some embodiments, ejection fraction is increased by about 6-12, 12-18, or 19-25% In some embodiments, ejection fraction is increased by about 5-20%, including 6, 7, 8, 9, 10, 11, 12, 13, 14, 15, 16, 17, 18, and 19%.

In several embodiments, the removal of one or more subpopulations of cells results (including but not limited to those expressing c-kit) in tissue regeneration after administration of the remaining therapeutic cells. In several embodiments, the tissue regeneration is manifest as an increase in the viability of the diseased or damaged tissue. In several embodiments the increase in viability is a result of increased cell division, while in other embodiments the increase is viability is due to a decrease in cell death (e.g., apoptosis or necrosis). In several embodiments, combinations of such effects results in tissue regeneration. As a non-limiting example, administration of a population of therapeutic cells that has been depleted (partially or completely) of certain subpopulations of stem cells (e.g., c-kit positive and/or CD90 positive cells) to a subject have suffered a myocardial infarction results in a reduction in infarct size. In some embodiments, infarct size is reduced by about 5%. In some embodiments, infarct size is reduced by about 10%. In some embodiments, infarct size is reduced by about 15%. In some embodiments, infarct size is reduced by about 1-3, 4-7, or 7-11%. In some embodiments, infarct size is reduced by 5-15%, including 6, 7, 8, 9, 10, 11, 12, 13, and 14%. In several embodiments, the reduction in infarct size ameliorates cardiac function (e.g., there is more cardiac tissue present that is capable of functioning at a level close to normal).

In several embodiments, the removal of one or more subpopulations of cells (including but not limited to those expressing c-kit) results in reduced risk of teratoma formation after administration of the remaining therapeutic cells to a subject. Certain subpopulations of cells that are particularly "stem" in nature may results in unwanted differentiation and growth into cell types that are distinct (and in some cases not functionally complementary) to the target tissue. Teratoma formation is thus a potential concern with certain types of cell therapy. In some cases, teratoma formation results from the potency (e.g., sternness) of a cell, and/or from a profile of paracrine modulators that is generated by such cells. Thus, in several embodiments, the removal of such subpopulations reduces the risk that other cells (either administered or endogenous) respond to such signals in a manner resulting in teratoma formation.

In several embodiments, the removal of one or more subpopulations of cells results in an increased uniformity of remaining therapeutic cells to be administered to a subject. In several embodiments, this increased uniformity leads to reduction in unexpected adverse events (e.g., teratoma formation, immune response) as the administered population can be more specifically defined prior to administration. In several embodiments, the increased uniformity leads to a reduction in conflicting signals that the administered population of therapeutic cells generates. For example, in some embodiments, the administration of a therapeutic cell population comprising two or more types of cells may be less therapeutically effective because the cells of a first type generate a signal or therapeutic effect that potentiates the effect of the cells of a second type. Of course, in several embodiments, the inclusion of two or more types of cells in the administered therapeutic cells leads to synergistic effects as compared to administration of any of the individual types.

In several embodiments, the removal of one or more subpopulations of cells results in an improved engraftment of the administered cells in the target tissue of a subject. In several embodiments, engraftment of cells in the target tissue provides a direct regenerative effect on the target tissue (e.g., the damaged or diseased cells are directly replaced by the administered cells). In some embodiments, engraftment of the administered therapeutic cells further reduces the risk of teratoma formation, at least in part due to the retention of the cells at the desired target site. While in some embodiments, the efficiency of engraftment is improved; in some embodiments the duration of engraftment is coordinately increased (e.g., duration of engraftment increased from time frames on the order of days or a few weeks to several weeks or even months). However, in some embodiments, long-term engraftment is not required for beneficial therapeutic effects to be realized. In several embodiments, beneficial therapeutic effects are realized, at least in part, due to indirect effects of the administered cells. For example, in several embodiments, the administered therapeutic cells release one or more paracrine factors that induce local or remote regenerative effects without directly replacing the damaged or diseased cells. The released paracrine factors may, in several embodiments, stimulate endogenous cells to initiate self-repair mechanisms and/or anti-apoptosis pathways. In several embodiments, the released paracrine factors function to signal endogenous cells at a remote location to initiate reparative effects and/or migrate to the site of damage and replace the damaged cells. In several embodiments, the paracrine factors comprise growth factors and/or cytokines (or other releasable factors). In several embodiments, the paracrine factors comprise one or more growth factors or cytokines selected from the group consisting of: ENA-78, G-CSF, GM-CSF, GRO, GRO-alpha, 1-309, IL-1 alpha, IL-1 beta, IL-2, IL-3, IL-4, IL-5, IL-6, IL-7, IL-8, IL-10, IL-12, IL-13, IL-15, interferon gamma, MCP-1, MCP-2, MCP-3, M-CSF, MDC, MIG, MIP-1 beta, MIP-1 delta, RANTES, SCF, SDF-1, TGF-beta 1, TNF-beta, EGF, IGF-1, angiogenin, oncostatin M, thrombopoeitin, VEGF, PDGF-BB, leptin, BDNF, BLC, Ck beta 8-1, eotaxin, eotaxin-2, eotaxin-3, FGF-4, FGF-6, FGF-7, Flt-3 ligand, fractalkine, GCP-2, GDNF, HGF, IGFBP-1, IGFBP-2, IGFBP-3, IGFBP-4, IL-16, IP-10, LIF, LIGHT, MCP-4, MIP-3 alpha, NAP-2, NT-3, NT-4, osteopontin, osteoprogenerin, PARC, PIGF, ST2, TGF beta 2, TGF beta 3, TIMP-1 and TIMP-2. In several embodiments, the growth factors or cytokines comprise one or more of VEGF, HGF, and IGFI. Combinations are used in several embodiments.

In several embodiments, the removal of one or more subpopulations of cells results in a higher degree of uniformity of the paracrine factors released. In such embodiments, the greater degree of uniformity may result is a reduction in signaling through counterproductive pathways that are initiated as result of the administration of the therapeutic cells. For example, the subpopulation of removed cells may generate a paracrine signal that initiates a pathway leading to cell death, whereas the remainder of the therapeutic cells has signals related to anti-apoptotic pathways. Thus, the removal of that subpopulation acts, in some embodiments, as a removal of an inhibition of the positive therapeutic benefits of the administered cells.

In several embodiments, the removal of one or more subpopulations of cells results in an increased variety of choices for delivery routes. In several embodiments, the removed subpopulation may be of a different size, have a different propensity for agglutination, and the like. The removal of the subpopulation, in several embodiments, results in a more uniform population of therapeutic cells, which may reduce concerns about potential adverse effects during administration. For example, if the removed subpopulation is larger in size that the remaining therapeutic cells, administration that results in passage of the therapeutic cells through smaller arterioles/venules presents a reduced concern of blockage of those arterioles/venules (because larger cells are removed). In several embodiments, the therapeutic cells populations ranging from about 50-200 µm in diameter are used. For example, in one embodiment for cardiac repair, cardiospheres having a size of about 50-150 µm in diameter are used. In other cardiac treatment embodiments, CDCs are used, which is particularly advantageous due to the size of CDCs being less than that of cardiospheres. In some embodiments, CDCs are of a size that is associated with little to no risk of embolization of at the arteriolar level. For example, in several embodiments, CDCs are less than about 50 µm in diameter. In some embodiments, CDCs are less than about 40 µm in diameter, less than about 30 µm in diameter, less than about 20 µm in diameter, and less than about 10 µm in diameter. In some embodiments, CDCs range from about 5 to about 10 µm in diameter, about 6 to about 11 µm in diameter, about 7 to about 12 µm in diameter, about 8 to about 13 µm in diameter, about 9 to about 14 µm in diameter, about 10 to about 15 µm in diameter, about 11 to about 16 µm in diameter, about 12 to about 17 µm in diameter, about 13 to about 18 µm in diameter, about 14 to about 19 µm in diameter, about 15 to about 20 µm in diameter, about 20 to about 25 µm in diameter, about 25 to about 30 µm in diameter, about 30 to about 35 µm in diameter and overlapping ranges thereof. In some embodiments, CDCs are less than about 75% of the size of a cardiosphere. In some embodiments, CDCs are less than about 50% of the size of a cardiosphere. In some embodiments, CDCs are less than about 25% of the size of a cardiosphere. In some embodiments, CDCs range from about 75% to about 70% of the size of a cardiosphere, from about 70% to about 65% of the size of a cardiosphere, from about 65% to about 60% of the size of a cardiosphere, from about 60% to about 55% of the size of a cardiosphere, from about 55% to about 50% of the size of a cardiosphere, from about 50% to about 45% of the size of a cardiosphere, from about 45% to about 40% of the size of a cardiosphere, from about 40% to about 35% of the size of a cardiosphere, from about 35% to about 30% of the size of a cardiosphere, from about 30% to about 25% of the size of a cardiosphere, from about 25% to about 20% of the size of a cardiosphere, from about 20% to about 15% of the size of a cardiosphere, from about 15% to about 10% of the size of a cardiosphere, from about 10% to about 5% of the size of a cardiosphere, from about 5% to about 1% of the size of a cardiosphere, and overlapping ranges thereof.

In several embodiments, the removal of one or more subpopulations of cells (including but not limited to those expressing c-kit) results in a reduction of destruction (or other varieties of removal or inactivation) of the administered cells. In several embodiments, the removed subpopulation may bear a marker, or result in activation of a signaling cascade, that induces endogenous mechanisms to destroy or inactivate the administered cells. Thus, in some embodiments, without that subpopulation (or subpopulations), such signals are reduced or non-existent. In several embodiments, these effects ameliorate the engraftment of the cells. However, in some embodiments, the administered cells are destroyed (or inactivated) by the endogenous mechanisms on the same or similar time-frame, regardless of the absence of that subpopulation (or subpopulations).

Similarly, in several embodiments, the removal of one or more subpopulations of cells (including but not limited to those expressing c-kit) results in an increased viability of administered therapeutic cells. In several embodiments, this is due to a reduction in signals or actions by endogenous mechanisms that act against the administered cells. In other embodiments, the removal of the subpopulation removes a direct negative influence that the subpopulation may have had on the remaining administered therapeutic cells.

Likewise, in several embodiments, the removal of one or more subpopulations of cells (including but not limited to those expressing c-kit) results in an increased viability of endogenous cells. In several embodiments, this occurs for similar reasons as discussed above, e.g., a removal of a negative factor or signal that the removed subpopulation would have otherwise provided. In several embodiments, the increased survival is due to reduction in apoptosis of the endogenous cells, increased division of the endogenous cells, or combinations thereof.

In several embodiments, the removal of one or more subpopulations of cells results in increased vascularization of the target tissue. In certain embodiments, this is of particular importance to the resultant beneficial effects, as the initial damage or disease may have been the result of inadequate blood or oxygen supply (e.g., in the case of myocardial infarction).

In some embodiments, the removal of one or more subpopulations allows for the generation of a specifically tailored therapeutic cell population. For example, if a particular subject has been subject to a specific type of injury or disease, which presents a known panel of symptoms, selective removal of various subpopulations can be performed to generate a population of therapeutic cells designed to specifically address that disease or injury, or the associated symptoms. In several embodiments, tailored cell populations can be generated by combining populations from multiple tissue sources (e.g., liver and cardiac) in order to develop a therapeutic population which results in a specifically desired set of beneficial effects. In several embodiments, these "tailored" populations of therapeutic cells operate synergistically to provide a greater degree of tissue repair and/or regeneration than non-tailored populations.

### Methods of Treatment

In several embodiments of the disclosure, the compositions disclosed herein are used for the treatment of a variety of diseases or injuries. For example, therapeutic cell populations depleted of one or more subpopulations of cells that are generated according to the methods can be used to treat cardiac disease or injury (including, but not limited to, congestive heart failure, myocardial infarction, and chronic ischemia), spinal cord injury, traumatic brain injury. The therapeutic cell compositions disclosed herein can also be used, in several embodiments, to treat the damage to cells or tissues that results from secondary effects of injury such as post-injury inflammation, infection, auto-digestion (for example, by proteases liberated as a result of an injury or trauma).

In several embodiments, the methods and compositions disclosed herein can be used to treat chronic diseases, including but not limited to neurological impairments or neurodegenerative disorders (e.g., , Alzheimer's disease, Parkinson's disease, Huntington's disease, epilepsy, dopaminergic impairment, dementia resulting from other causes such as AIDS, multiple sclerosis, amyotrophic lateral sclerosis, cerebral ischemia, physical trauma any other acute injury or insult producing neurodegeneration), immune deficiencies, repopulation of bone marrow (e.g., after bone marrow ablation or transplantation), arthritis, auto-immune disorders, inflammatory bowel disease, cancer, diabetes, muscle weakness (e.g., muscular dystrophy, amyotrophic lateral sclerosis, and the like), progressive blindness (e.g. macular degeneration), and progressive hearing loss.

In several embodiments, the therapeutic cell populations can be administered to subject to treat a variety of cancers, including but not limited to acute lymphoblastic leukemia (ALL), acute myeloid leukemia (AML), adrenocortical carcinoma, kaposi sarcoma, lymphoma, gastrointestinal cancer, appendix cancer, central nervous system cancer, basal cell carcinoma, bile duct cancer, bladder cancer, bone cancer, brain tumors (including but not limited to astrocytomas, spinal cord tumors, brain stem glioma, craniopharyngioma, ependymoblastoma, ependymoma, medulloblastoma, medulloepithelioma, breast cancer, bronchial tumors, burkitt lymphoma, cervical cancer, colon cancer, chronic lymphocytic leukemia (CLL), chronic myelogenous leukemia (CML), chronic myeloproliferative disorders, ductal carcinoma, endometrial cancer, esophageal cancer, gastric cancer, Hodgkin lymphoma, non-Hodgkin lymphoma hairy cell leukemia, renal cell cancer, leukemia, oral cancer, liver cancer, lung cancer, lymphoma, melanoma, ocular cancer, ovarian cancer, pancreatic cancer, prostate cancer, pituitary cancer, uterine cancer, and vaginal cancer.

In several embodiments, the therapeutic cell populations that have had one or more subpopulations removed are used to treat target tissues that are infected, for example with one or more bacteria, viruses, fungi, and/or parasites. In several embodiments, the therapeutic cell populations that have had one or more subpopulations removed are used to treat tissues with infections of bacterial origin (e.g., infectious bacteria is selected the group of genera consisting of *Bordetella, Borrelia, Brucella, Campylobacter, Chlamydia* and *Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema, Vibrio,* and *Yersinia,* and mutants or combinations thereof). In several embodiments, the therapeutic cells inhibit or prevent one or more bacterial functions, thereby reducing the severity and/or duration of an infection. In several embodiments, administration of the therapeutic cells sensitizes the bacteria (or other pathogen) to an adjunct therapy (e.g., an antibiotic).

In several embodiments, the therapeutic cell populations that have had one or more subpopulations removed are used to treat viral infections, such as those caused by one or more viruses selected from the group consisting of adenovirus, Coxsackievirus, Epstein-Barr virus, hepatitis a virus, hepatitis b virus, hepatitis c virus, herpes simplex virus, type 1, herpes simplex virus, type 2, cytomegalovirus, ebola virus, human herpesvirus, type 8, HIV, influenza virus, measles virus, mumps virus, human papillomavirus, parainfluenza virus, poliovirus, rabies virus, respiratory syncytial virus, rubella virus, and varicella-zoster virus.

Various routes of administration may be used, depending on the embodiment. For example, depending on the target tissue, a particular route of administration may be preferred over another, as the preferred route is likely to allow increased delivery of the therapeutic cells. However, in some embodiments, a route that is likely to allow increased delivery of the therapeutic cells may not be chosen, but rather a route of administration that is less invasive may be preferred (for example, in those patients whose health status may not tolerate a more invasive route).

In several embodiments, the therapeutic cells are delivered locally. Local administration comprises, in several embodiments, direct injection (or other delivery method) to the target tissue. Catheters are used for direct delivery to certain tissues with a reduced degree of invasiveness, in several embodiments. Intramuscular delivery is also used in some embodiments. Lavage, perfusion, or infusion into the target tissue is also used in several embodiments. In several embodiments, systemic delivery is employed. Therapeutic cells are delivered, depending on the embodiment, intravenously or intrarterially. In several embodiments for treatment of cardiac disease or injury, intracoronary, or intramyocardial routes (or other routes) of administration are used. Advantageously, in several embodiments, the removal of one or more subpopulations of cells improves the migration of therapeutic cells to a target tissue, thus improving the delivery efficiency when systemic routes of administration are used.

The dose of therapeutic cells delivered may vary, depending on the embodiment. In several embodiments, doses between about 1 x 10⁵ cells and 1 x 10⁹ cells are administered. In several embodiments, doses may range from about 1 x 10⁵ cells to about 1 x 10⁶ cells, from about 1 x 10⁶ cells to about 1 x 10⁷ cells, from about 1 x 10⁷ cells to about 1 x 10⁸ cells, from about 1x10⁸ cells to about 1 x 10⁹ cells, and overlapping ranges thereof. In several embodiments, the dose of cells is determined based on the body weight of the recipient (e.g., on a per kilogram basis). Thus, in some embodiments, about 1 x 10⁴ to about 1 x 10⁸ cells per kilogram of body weight of the subject are administered, including about 1 x 10⁴ to about 1 x 10⁵ cells/kg, about 1 x 10⁵ to about 1 x 10⁶ cells/kg, about 1 x 10⁶ to about 1 x 10⁷ cells/kg, about 1 x 10⁷ to about 1 x 10⁸ cells/kg, and overlapping doses within those ranges.

### EXAMPLES

Examples provided below are intended to be non-limiting embodiments of the invention.

### Example 1 - Regenerative Potential of Cardiosphere-Derived Cells (CDCs) and c-kit^{DEP} Cardiosphere-Derived Cells in an Animal Model

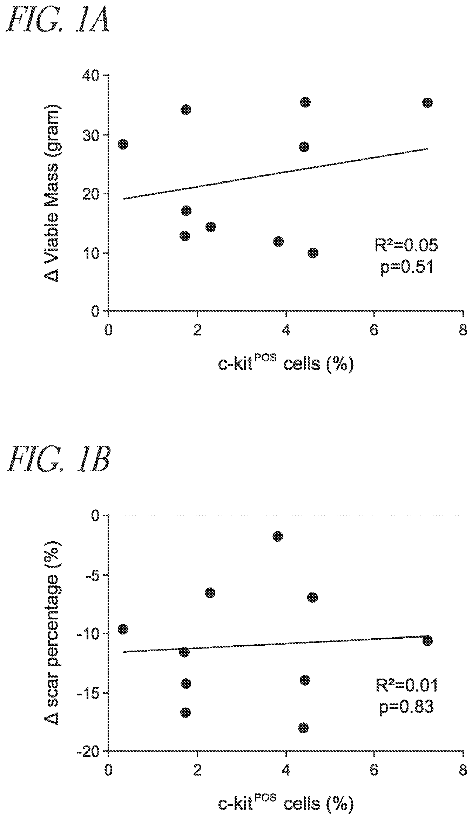

As discussed above, in several embodiments, cell populations generated from a tissue source may comprise a plurality of subpopulations. In several embodiments, specific depletion (partial or complete) of one or more such subpopulations results in one or more positive benefits, and in many cases, no reduction in efficacy. As an example, a retrospective analysis of data from human patients in the recently completed CADUCEUS clinical trial suggests that there is little correlation between the percentage of c-kit^{POS} cells in a population of administered CDCs and the eventual regenerative potency of a population of CDCs (at least as evaluated by scar percentage and mass of viable heart tissue) (Figs. 1A-1B). In some cases, the presence of c-kit^{POS} cells in an administered population may lead to functional, anatomical, or other benefits to the recipient.

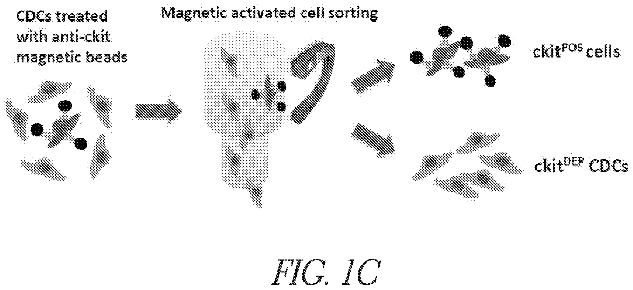

To further investigate the therapeutic efficacy of therapeutic cells depleted of selected subpopulations of cells, the following examples compared the effects of human CDCs with a population of human c-kit^{DEP} CDCs (e.g., a CDC population depleted of c-kit^{POS} cells). One embodiment of a method for depleting a population of therapeutic cells of a subpopulation is schematically shown in Figure 1C. An established mouse model of myocardial infarction was used to compare the regenerative potential of CDCs and c-kit^{DEP} CDCs . In brief, myocardial infarction (MI) was induced in SCID mice (n=6-8 mice) by left anterior descending artery (LAD) ligation. A dose of 10⁵ CDCs or 10⁵ c-kit^{DEP} CDCs were injected intramyocardially in the MI border zone.

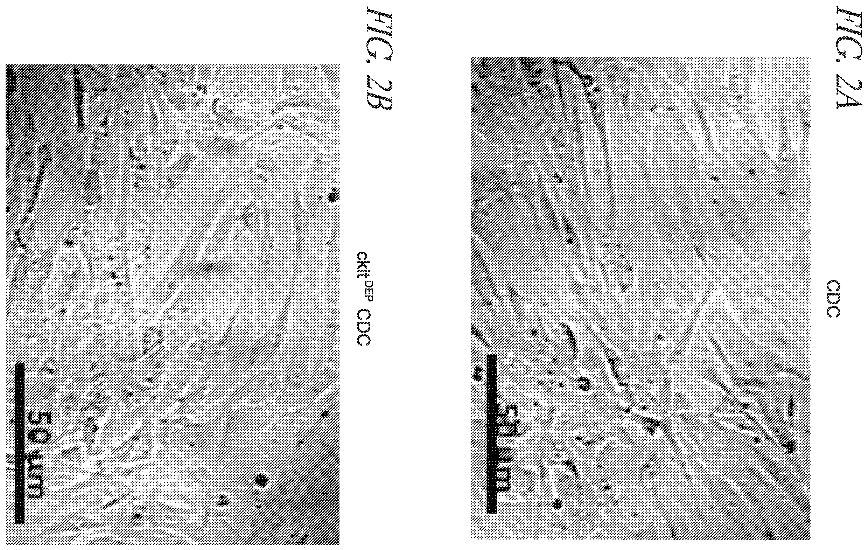

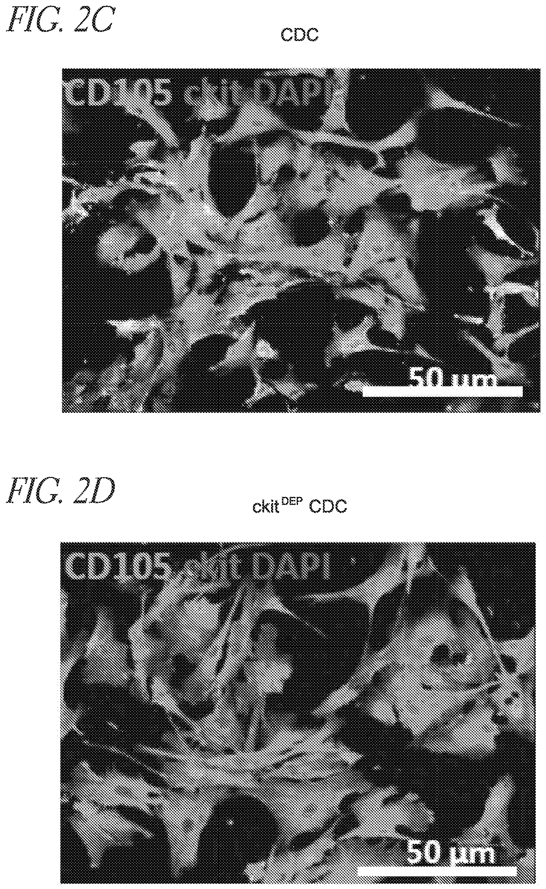

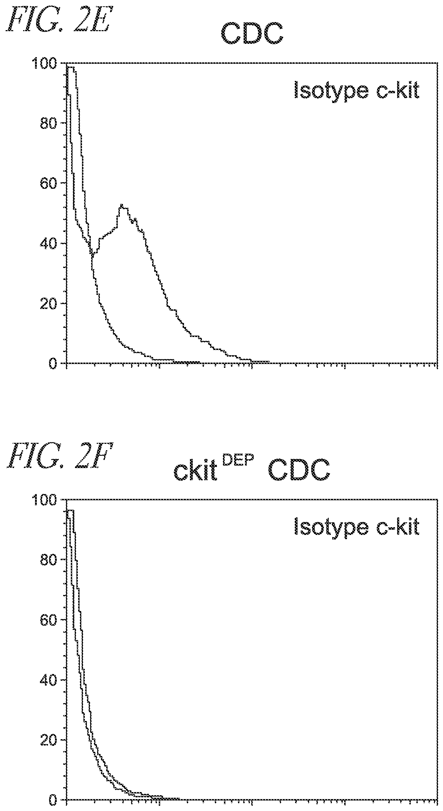

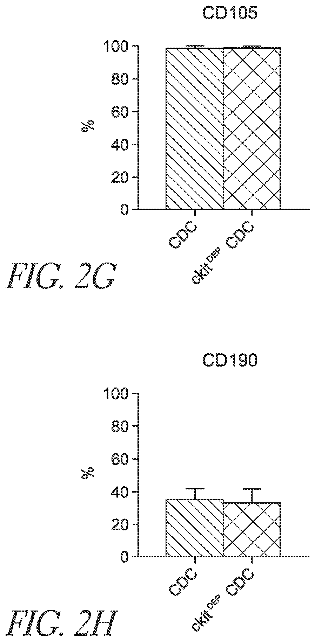

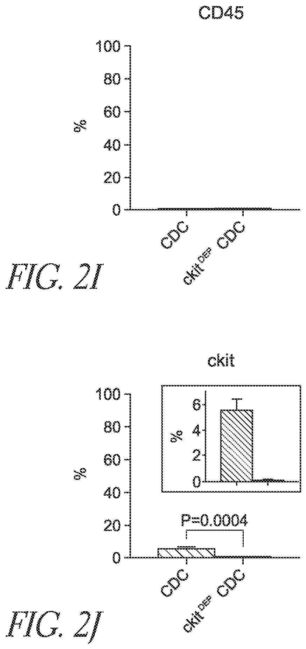

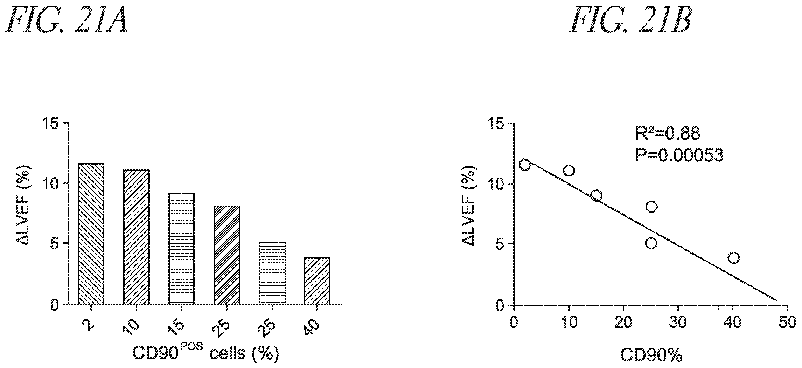

In this example, c-kit depletion was effected by magnetic activated cell sorting (MACS), but in several embodiments, such depletion can be achieved by one or more methods disclosed herein. Depending on the embodiment and the characteristics desired in the therapeutic cell population, depletion may be partial, substantially complete, or complete. In this example, cell morphology was examined using white light microscopy (Figures 2A-2B) and c-kit depletion was confirmed using confocal microscopy (Figures 2C-2D) and flow cytometry analysis (Figures 2E-2F). As can be seen in the data of Figure 2F, the percentage of c-kit-positive cells in the c-kit^{DEP} CDC population was nearly identical to the isotype control, suggesting that, in this embodiment, depletion was substantially complete (if not complete). Furthermore, to demonstrate the specificity of the depletion, the phenotype of the two populations (CDC and c-kit^{DEP} CDC) was investigated by analyzing the prevalence of other cell surface protein markers CD105, CD190, and CD45 (Figs. 2G-2I). The lack of significant differences between these markers on the CDC population as compared to the c-kit^{DEP} CDC indicates that the depletion was specific and did not affect other subpopulations. Moreover, these Figures are indicative of certain phenotypic characteristics of the CDCs, and those CDCs depleted of c-kit. For example, Figure 2G indicates that at least about 95% of CDCs and c-kit^{DEP} CDCs express CD105. In some embodiments, at least about 80%, at least about 85%, at least about 90%, at least about 95%, or more (and overlapping ranges therein) of c-kit^{DEP} CDCs express CD105. Figure 2H indicates that between about 20% to about 40% of c-kit^{DEP} CDCs express CD190. Figure 21 indicates that less than about 10% of c-kit^{DEP} CDCs express the CD45 marker. In several embodiments, less than about 20%, less than about 15%, less than about 10%, less than about 5%, less than about 2%, less than about 1%, or fewer (and overlapping ranges thereof) c-kit^{DEP} CDCs express CD45. This specificity was further verified by comparison of the c-kit expression levels between the CDC and c-kit^{DEP} CDC populations (Figure 2J), which shows a reduction in the amount of c-kit positive cells (as compared to the starting CDC population) after depletion was performed.

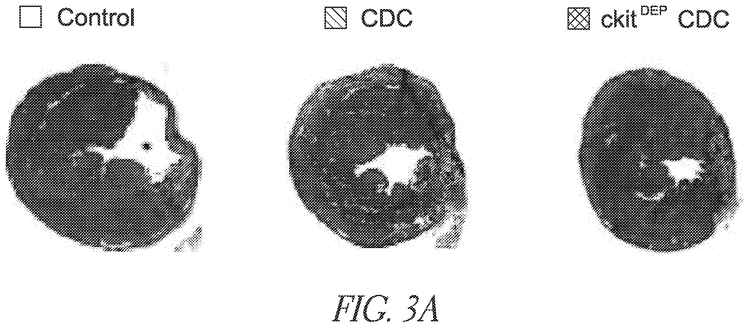

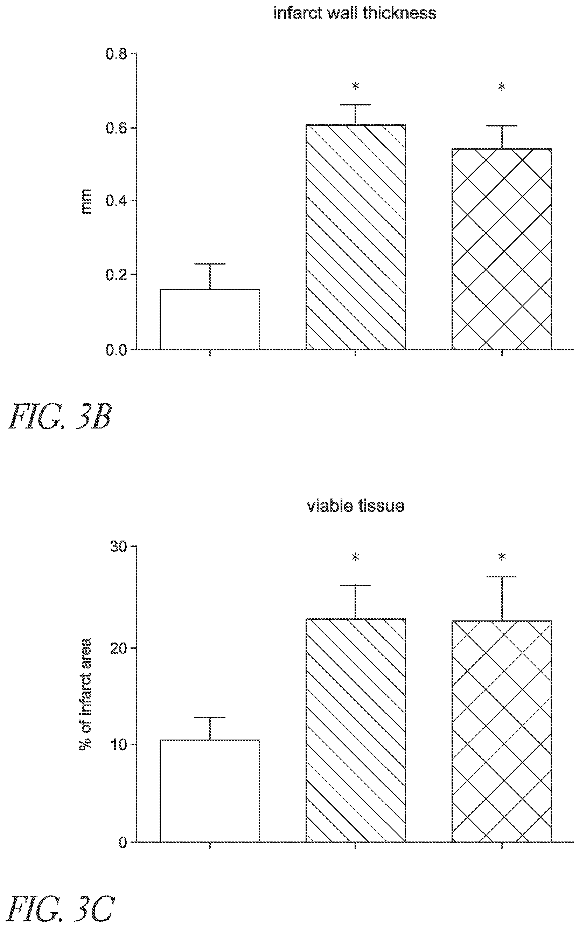

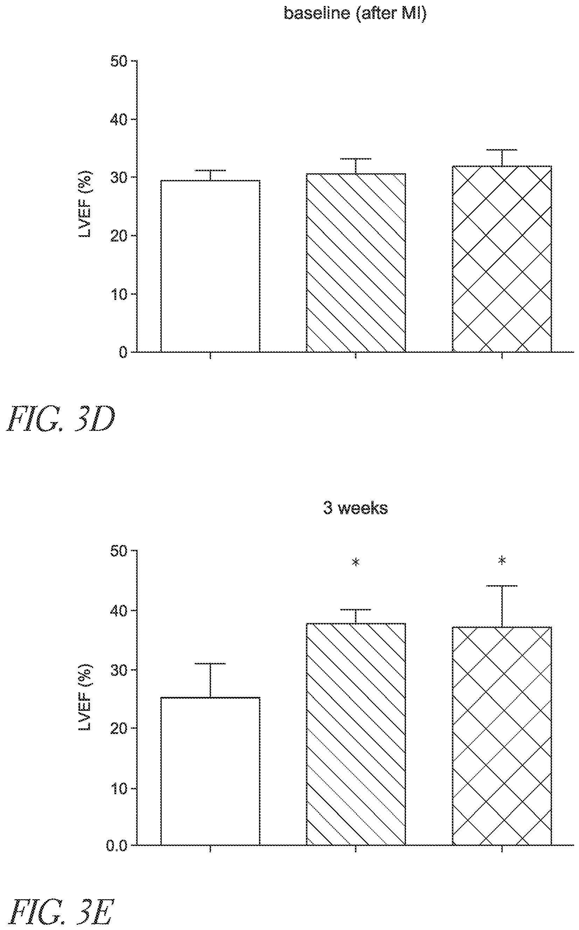

With respect to therapeutic benefit, administration of the c-kit^{DEP} CDC population resulted in similar therapeutic benefit with regard to regeneration of viable tissue and infarct wall thickness (Figures 3A-3C). Both populations increased the thickness of the heart wall post-infarct as well as the percentage of viable tissue in the infarct area. Furthermore, both CDCs and c-kit^{DEP} CDCs resulted in significant LV function augmentation as compared to control-treated hearts (Figures 3D-3E).

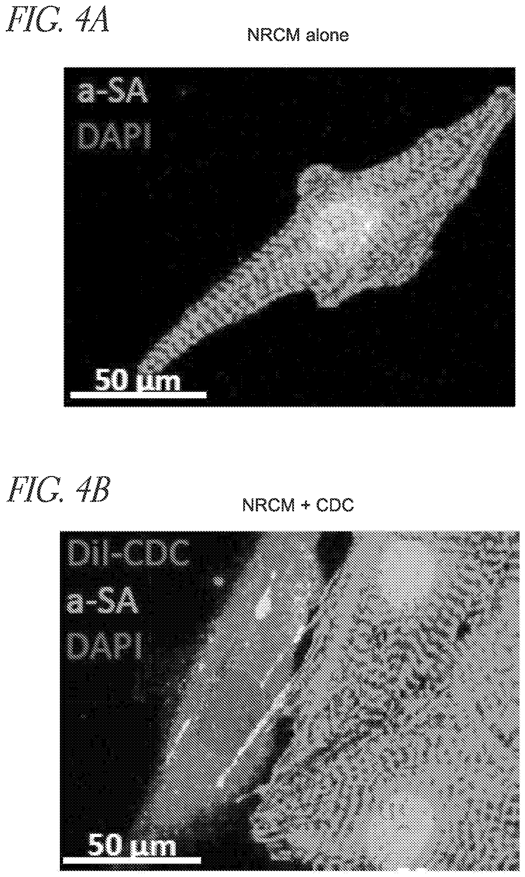

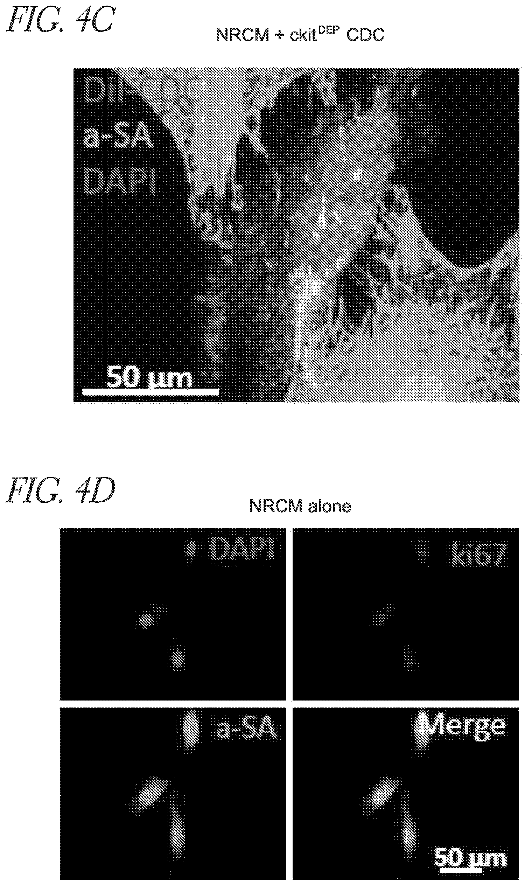

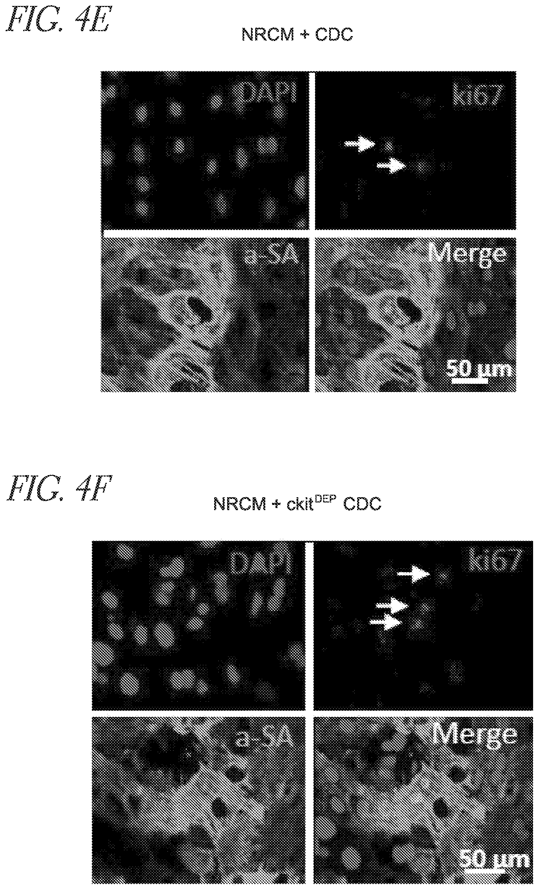

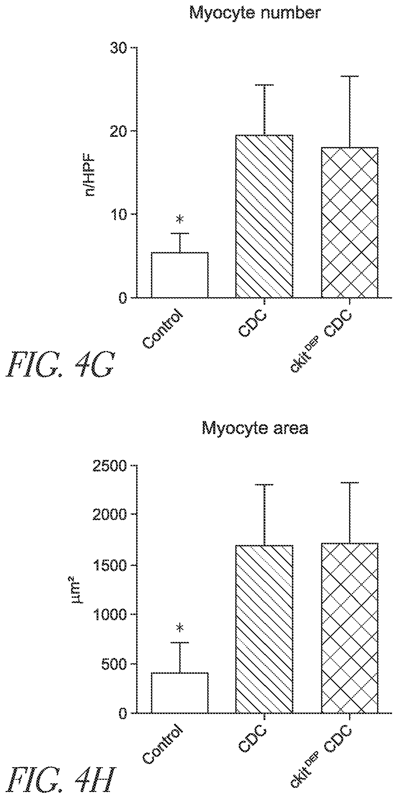

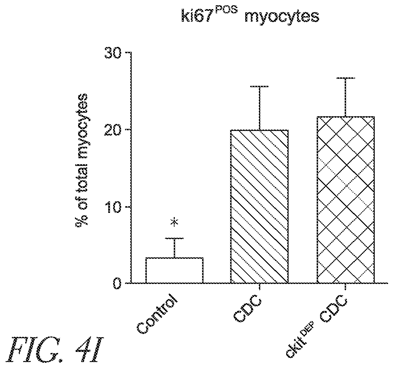

In an in vitro co-culture model (CDCs or c-kit^{DEP} CDCs were co-cultured with neonatal rat cardiomyocytes (NRCM)) to determine the ability of each population to promote cardiomyocyte function. Figures 4B-4C depict similar colocalization of CDCs and c-kit^{DEP} CDCs with NRCM (shown alone in Figure 4A) in culture. Figures 4D and 4E depict an analysis of the number cardiomyocytes entering the cell cycle (represented by expression of Ki67, positive cells indicated by arrows). Figures 4G-4I depict the analysis of myocytes number, myocyte area, and the number of Ki67 positive myocytes, respectively. In each case, the CDCs and c-kit^{DEP} CDCs resulted in significant increases as compared to control. This indicates that both cell populations are capable of promoting improvements in myocyte function. In addition, as discussed above, removal of one or more subpopulations may have additional positive benefits (e.g., reduced apoptosis that accompanies the increased cell division).

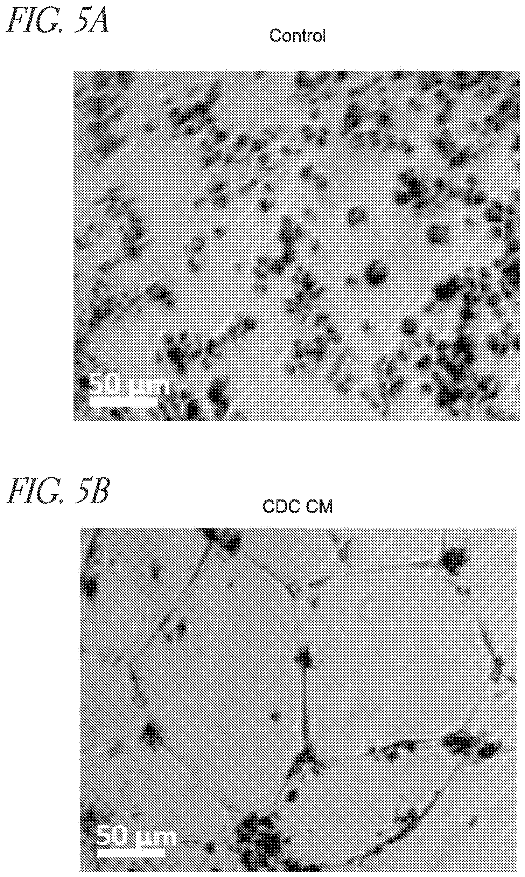

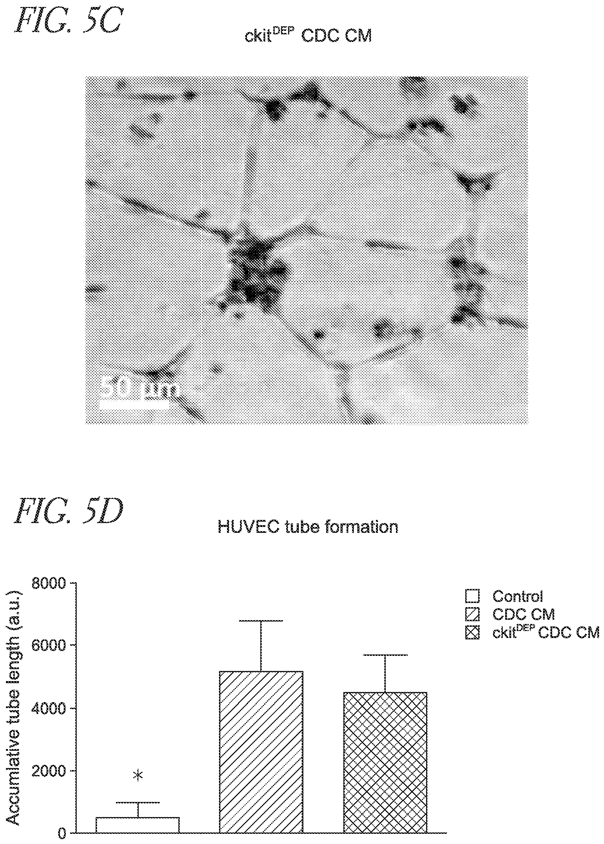

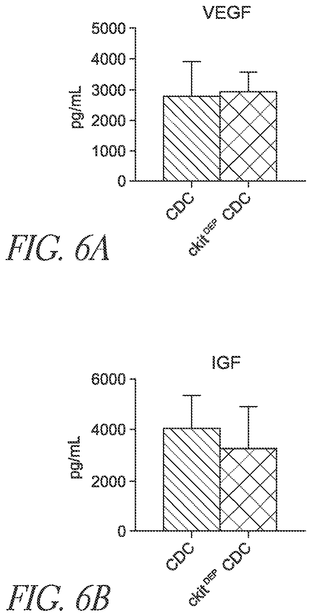

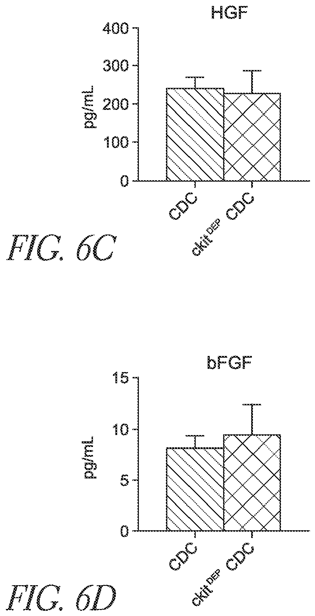

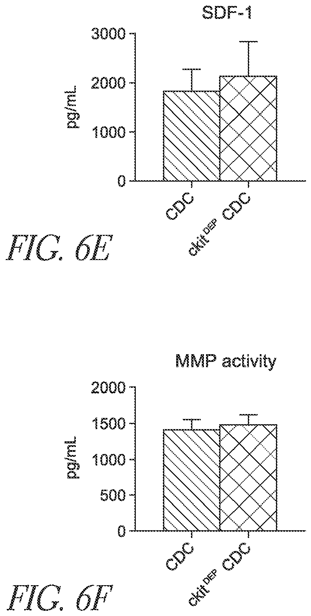

In additional studies, CDCs and c-kit^{DEP} CDCs promoted significantly greater endothelial cell tube formation on Matrigel compared to control (Figs. 5A-5D). Finally, both CDCs and c-kit^{DEP} CDCs produced similar amounts of certain paracrine factors VEGF, IGF, HGF, bFGF, SDF-1, and MMP activity (Figures 6A-6F). In other embodiments, however, the removal of one or more subpopulations results in a change in the paracrine profile that may result in one or more positive therapeutic benefits, as discussed above.

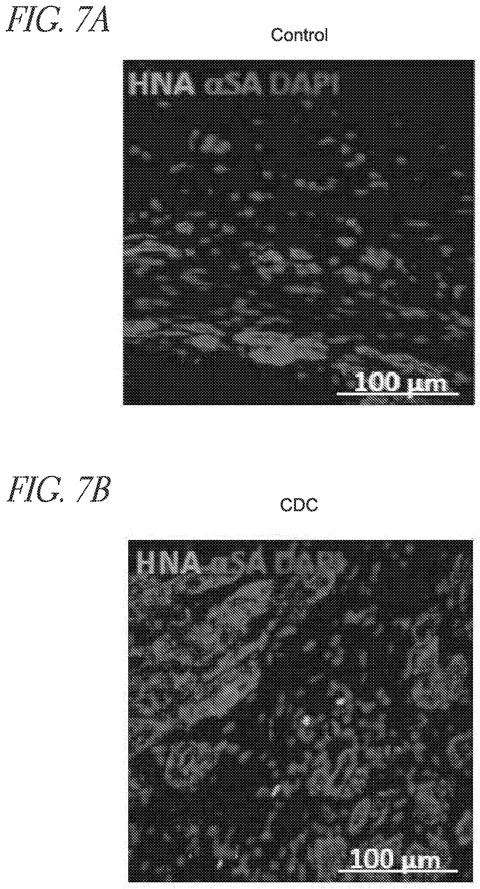

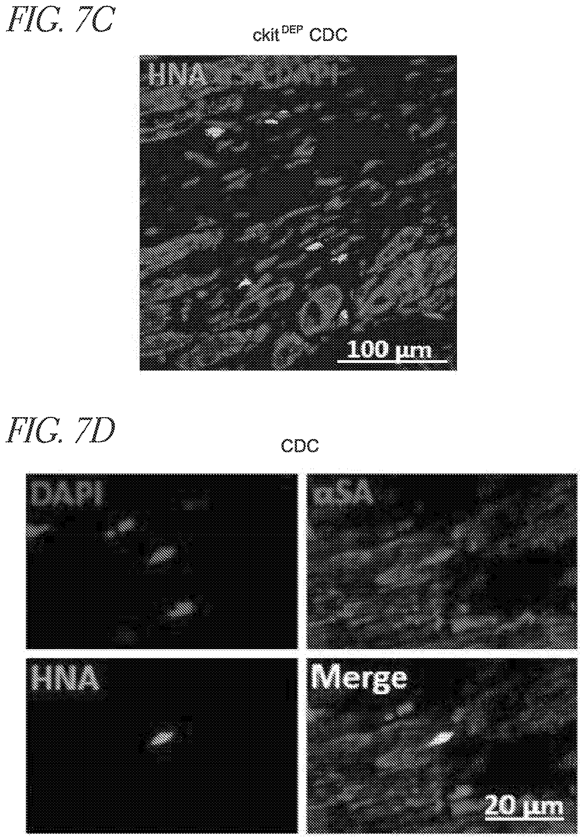

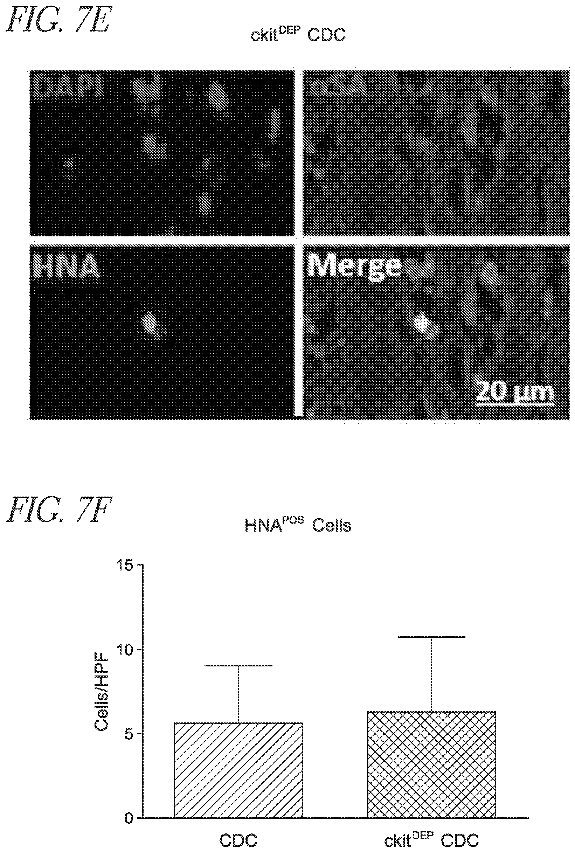

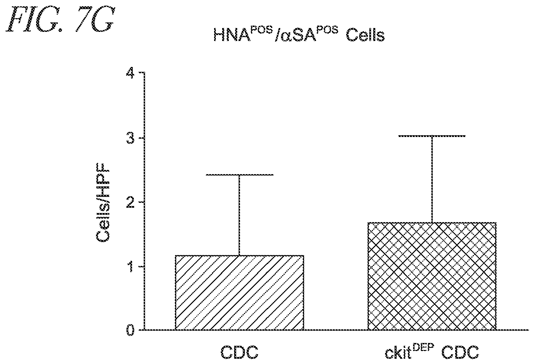

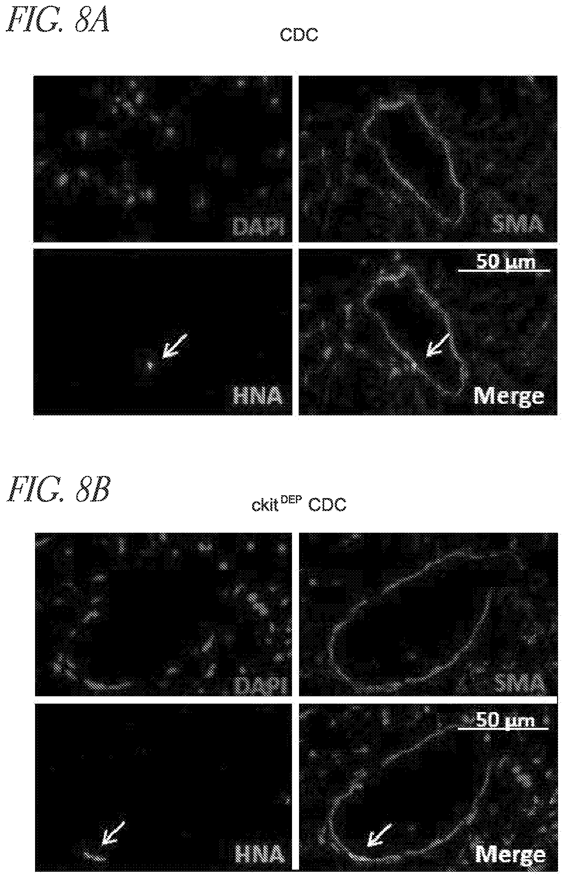

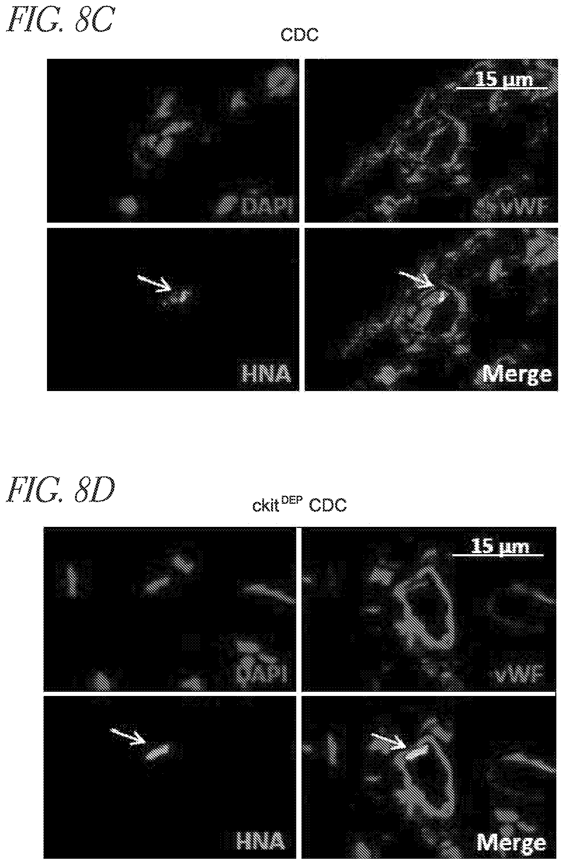

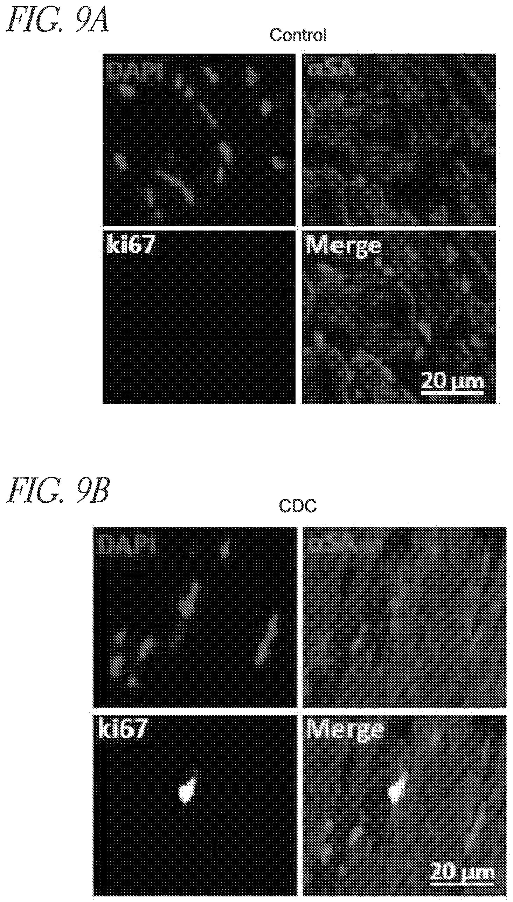

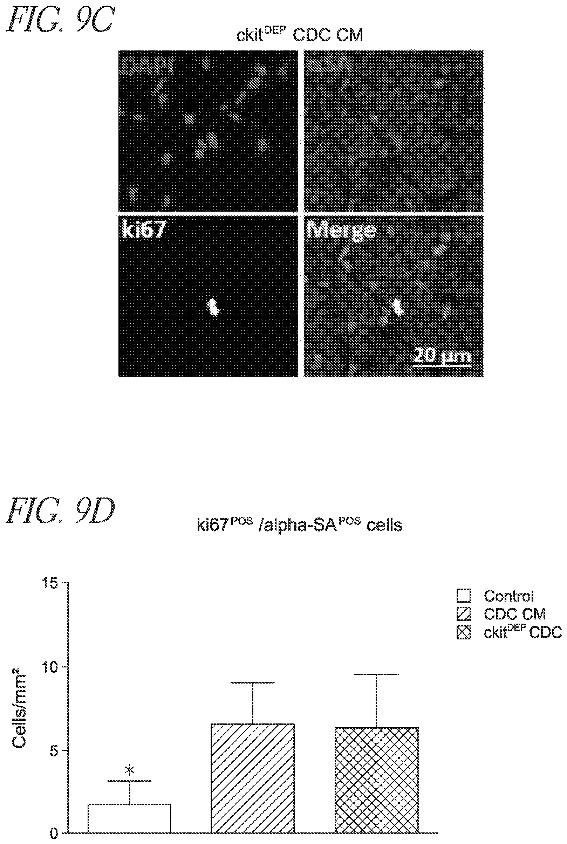

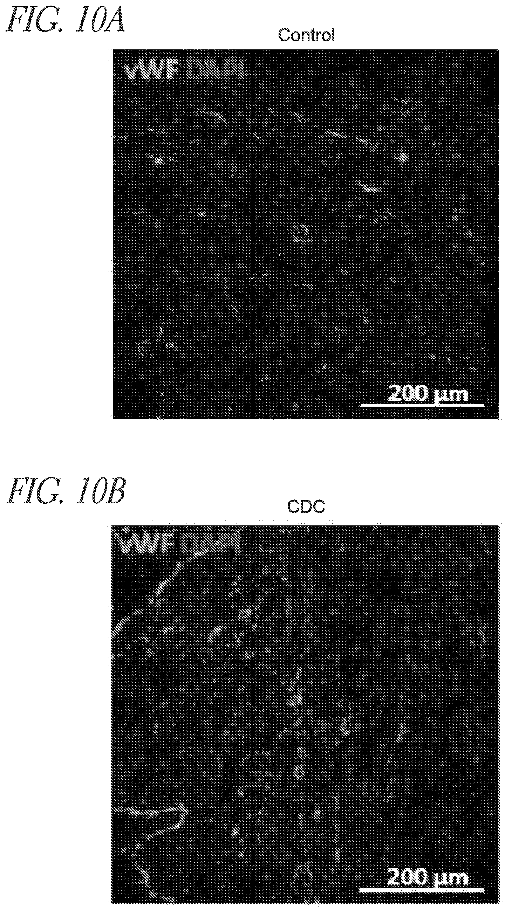

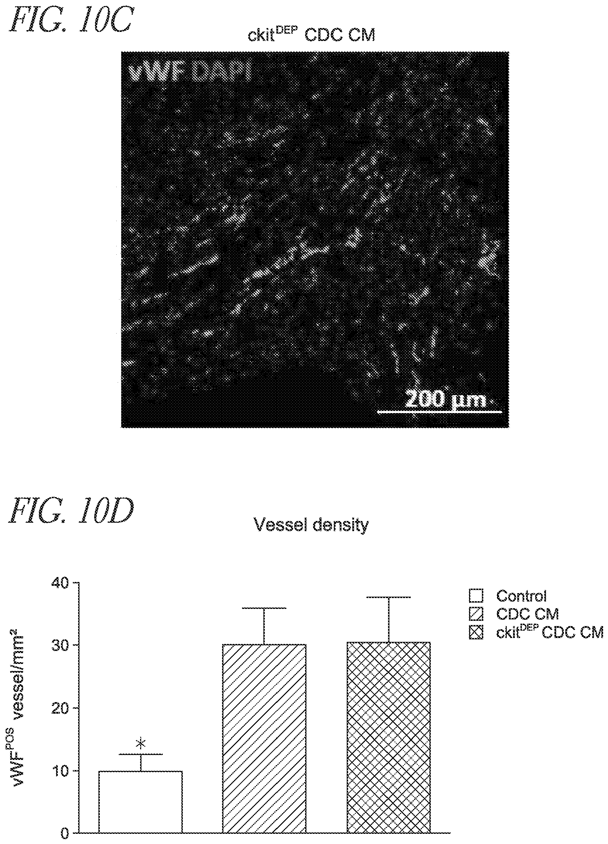

In in vivo studies, engraftment of the CDCs and c-kit^{DEP} CDCs after administration was compared. Figures 7A and 7C show data indicating that the depletion of c-kit does not significantly alter the ability of the c-kit^{DEP} CDCs from engrafting into the target tissue (as indicated by measuring the proportion of human nuclear antigen (HNA) positive cells). As discussed above, in several embodiments, the removal of one or more subpopulations may increase the duration of engraftment, reduce the potential for teratoma formation, and/or may enhance one or more other therapeutic aspects of the administered cells. Figures 7B and D indicate that there is no significant alteration in the ability of c-kit^{DEP} CDCs to differentiate into cardiomyocytes. Further supporting the in vitro data above, Figures 8B and 8D depict data that indicate that, as with CDCs, c-kit^{DEP} CDCs are capable of differentiating into both smooth muscle and endothelial cells after transplantation into post-MI mouse hearts. Likewise, depletion of c-kit did not alter the ability of the c-kit^{DEP} CDCs to promote cardiomyocyte cycling in vivo (as demonstrated by ki67 expression (Figures 9A-9D)) or angiogenesis (as demonstrated by vWF expression (Figs. 10A-10D)).

It shall be appreciated that these data indicate that, in several embodiments, CDCs having been depleted of one or more subpopulations of cells are therapeutically effective populations that can be used to effect repair or regeneration of damaged tissue (e.g., in a post-MI heart). In several embodiments, removal of certain subpopulations such as, for example, c-kit^{POS} are not associated with any decrease in the efficacy of the depleted CDC population. Thus, in several embodiments, c-kit^{DEP} CDCs are an effective population for repair or regeneration of tissue. In several embodiments this treatment could be used for tissue repair or regeneration in mammals. In several other embodiments this treatment could be used for tissue repair or regeneration in humans. In several embodiments, the tissue to be repaired or regenerated could include cardiac tissue, including but not limited to post-MI tissue. Moreover, as discussed above, in several embodiments the depletion (partial, substantial, or complete) of one or more subpopulations of cells results in one or more additional benefits that are associated with therapeutic efficacy (e.g., reduction in adverse events, more defined cell, and thus "tailorable" cell populations, improved cell viability). While these data investigated several indices of therapeutic benefit, additional advantages may exist when using c-kit^{DEP} CDCs that were not specifically investigated in this study (e.g., reduced apoptosis of administered and/or endogenous cells, enhanced direct regeneration, reduction of competing (e.g., self-limiting) pathways upon administration).

### Example 2 -Correlation of CD90 Expression and Scar Reduction Potency

Retrospective data analysis from the CADUCEUS trial indicates that a negative correlation exists between CD90 expression and scar reduction. In brief, the CADUCEUS trial involved harvesting of autologous heart cells from endomyocardial biopsy specimens which were then grown over a four-week period to a therapeutic dose. The trial population consisted of post-MI patients with left ventricular ejection fractions (LVEFs) between from 25% to 45% with subjects randomized 2:1 to receive CDC infusion compared to conventional medical therapy. Patients received either a CDC dose of 12.5 million cells or 25 million cells.

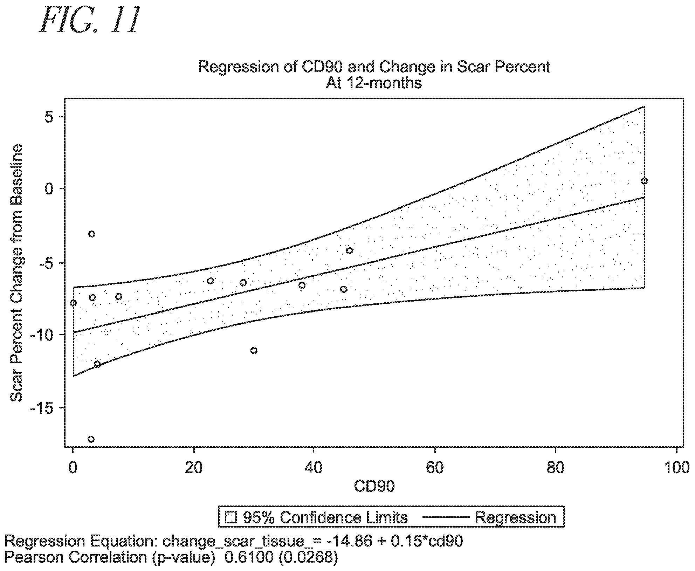

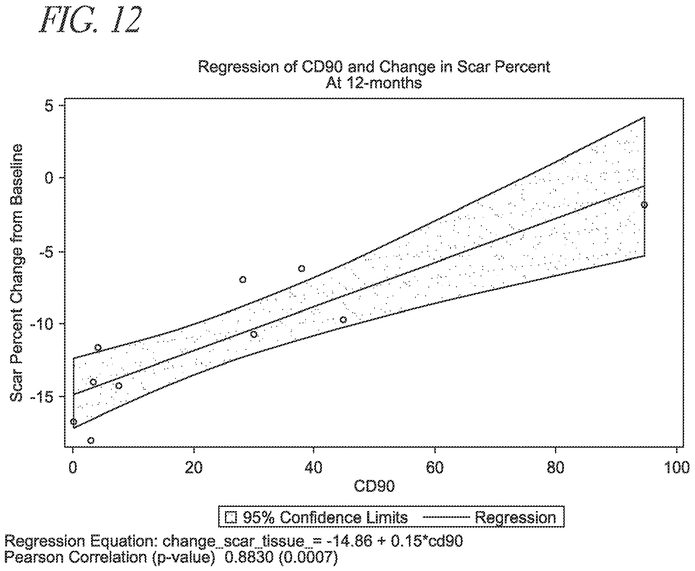

As discussed above, the depletion of c-kit-expressing CDCs from the harvested CDCs did not result in reduction in therapeutic efficacy (Figs. 1A-1B). Furthermore, retrospective analysis of CADUCEUS data indicates a negative correlation between CD90 expression in the CDC population and scar percentage at 6 and 12 months (Figs. 11, 12). In other words, CDC populations with less CD90-positive cells showed a higher scar reduction potency. These data indicate that, as discussed above, removal of certain subpopulations of cells, in several embodiments, has the capacity to increase one or more aspects of therapeutic efficacy.

It shall be appreciated that these data indicate that, subpopulations such as, for example, CD90^{POS} or c-kit^{POS} cells may be removed from the CDC population with coordinate improvements in therapeutic efficacy and/or other aspects of therapy generally (ease of administration, reduced adverse effects, less immune response, etc.). In several embodiments, c-kit^{DEP} CDCs are an effective population for regeneration. In several other embodiments, CD90^{DEP} CDCs are an effective population. In several embodiments, a population of CDCs is depleted of both c-kit and CD90. In several additional embodiments, cells exhibiting other cell surface markers are depleted from a CDC population, and result in an equally (if not more) effective population. Again, in several embodiments, the removal may not significantly alter the directly resulting therapeutic benefit (e.g., anatomy or function), but may improve one or more associated aspects of therapy that lead to an overall improved outcome for a patient in need of cell therapy.

### Example 3 - Further Characterization of the Depletion of CDCs of Specific Subpopulations

As discussed above, in several embodiments, depletion of CDCs of specific subpopulations does not alter the therapeutic potency of the remaining CDCs. Additional studies were performed as described in more detail below, to further elucidate what characteristics of CDCs are associated with (or change as a result of depletion of) specific subpopulations of CDCs.

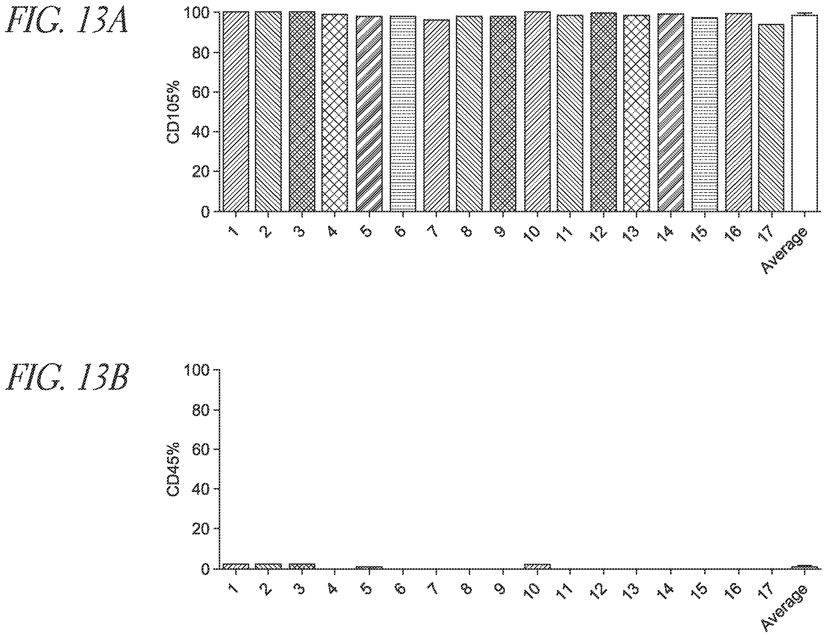

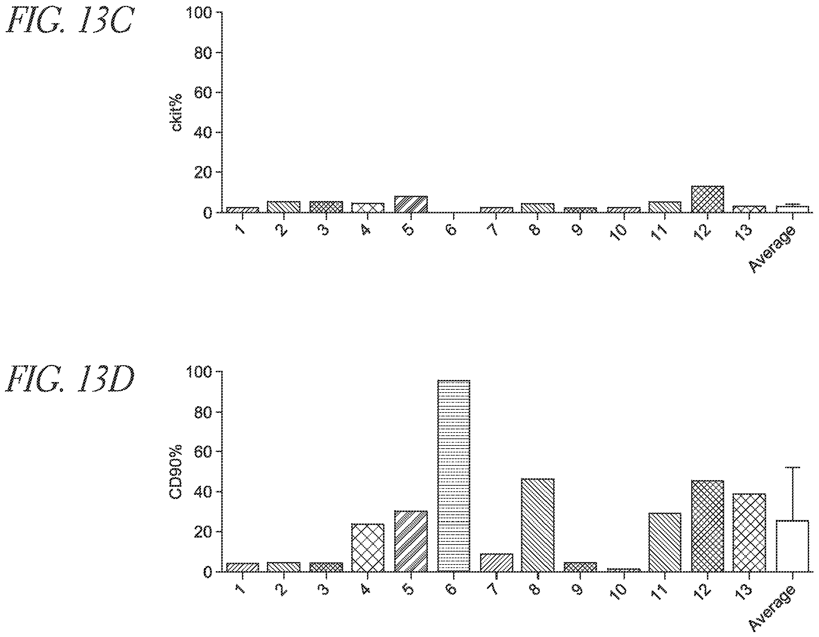

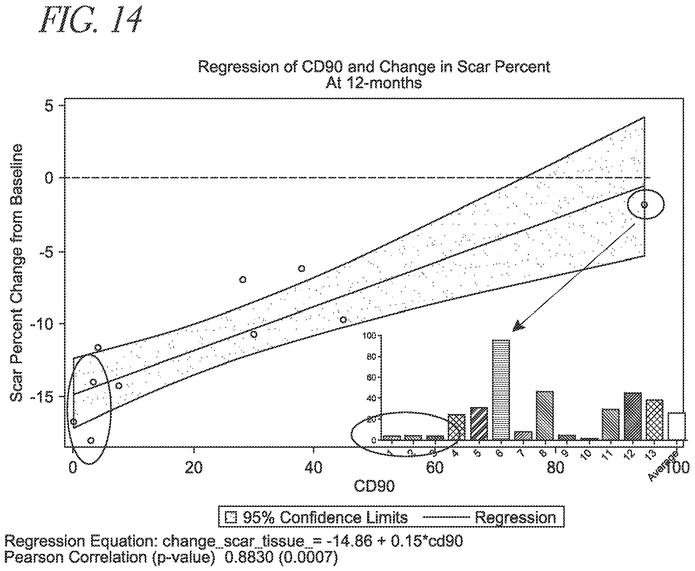

Expression of selected cell-surface markers was evaluated in seventeen individual CDC populations that were used clinically (in the CADUCEUS clinical trial). The average expression across all CDCs produced was also calculated. Figure 13A depicts CD105 expression, which is above about 90% in all CDC populations. CD105 is an established endothelial cell marker. In contrast Figure 13B depicts CD45 expression, which appears on less than about 5% of CDCs. CD45 is a tyrosine kinase that is expressed highly by hematopoietic cells. These data therefore suggest that CDCs are not cells derived from hematopoietic or bone marrow cells. The expression of ckit is shown in Figure 13C, and ranges from about 2% to about 7% of all CDCs. CD90 expression is shown in Figure 13D, with expression ranging widely, depending on the CDC preparation. About 90% of the CDCs of preparation 6 express CD90, while less than about 4% of the CDCs of preparation 10 express CD90. As discussed above (see Figures 1A-1B), there is no clinical correlation between ckit expression and the therapeutic outcome of CDC administration, at least as evaluated by change in viable mass or change in scar percentage. In contrast, greater CD90 positivity appears to be correlated with lesser therapeutic efficacy. As shown in Figure 14, three of the subjects showing the greatest reduction in scar percentage were treated with CDCs having low (e.g., less than about 5%) CD90 expression. In contrast, the subject treated with the CDCs of population 6 (over 90% CD90 positive CDCs) showed very little therapeutic improvement (at least as measured by reduction in scar size).

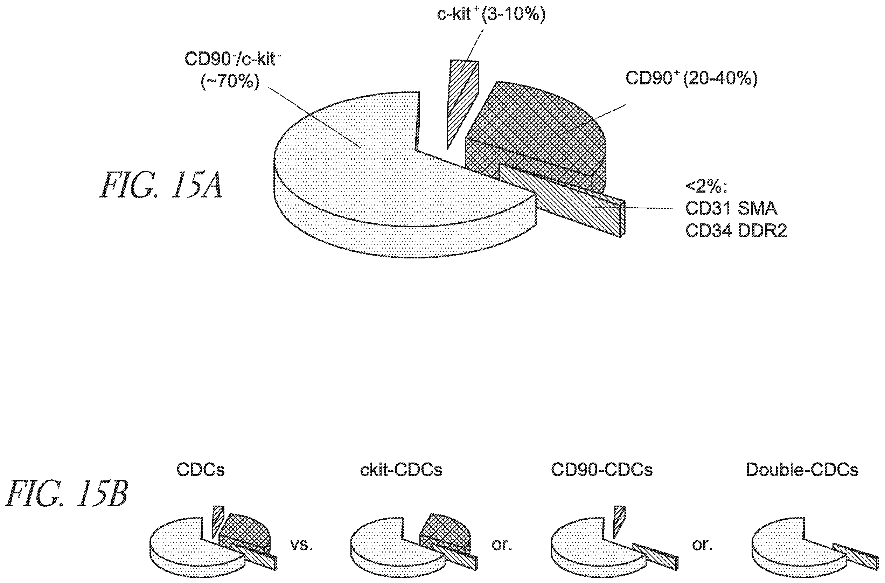

The average phenotypic composition of CDCs is schematically depicted in Figure 15A. To compare the therapeutic benefit of CD90-depleted and/or ckit-depleted CDCs to the non-depleted CDC population, the experiments described below were performed. The groups compared were as shown in Figure 15B, e.g., CDCs, ckit-depleted CDC, CD90 depleted CDCs, and CDCs depleted of both ckit and CD90 (also referred to a double-depleted CDCs).

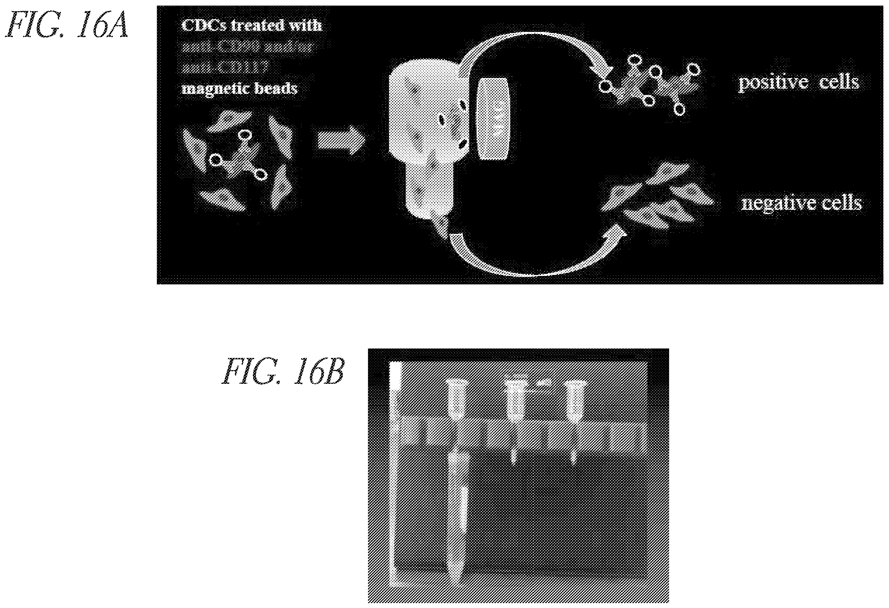

Figure 16A depicts a schematic of the methodology used for depleting the CDCs of the various subpopulations. CDCs, once generated according to the methods described above, are incubated with magnetic beads coupled to (i) an anti-CD90 antibody; (ii) an anti-ckit (also known as CD117) antibody, or (iii) an anti-CD90 antibody and an anti-ckit antibody. After incubation, the various CDC populations are passed through a conical funnel (see Figure 16B) adjacent to which a magnet has been placed. As the CDC populations pass through funnel, those that express CD90 and/or ckit will be retained within the funnel (e.g., there is a selecting out of the positive cells). The CDCs that do not interact with the magnetic beads will pass through and are collected for further characterization. These depletion methods are practiced using established methods (see e.g., Miltenyi Biotech and its MACS Cell Separation products and information).

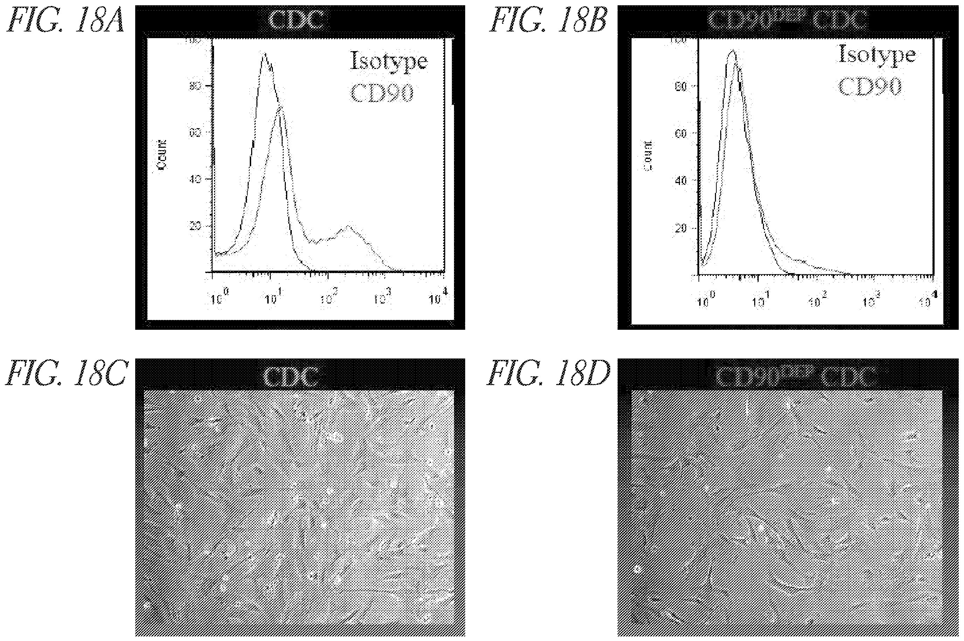

Healthy whole hearts from deceased donors were used to generate the CDCs for the experiments discussed herein (though as discussed above, portions of the heart, or even biopsies are used, depending on the embodiment). Because the average CD90 expression on CDCs used in the CADUCEUS clinical trial was -40%, two donor CDC populations that were -40% CD90-positive were used. After being processed by the depletion protocol, CD90 expression was evaluated. Figure 18A depicts flow cytometry analysis of non-depleted CDCs. As evidenced by the right-shifted signal curve, a CD90⁺ subpopulation was detected. Figure 18C depicts white light microscopy images of the non-depleted CDC population. Figure 18B shows flow cytometry data after the CD90 depletion protocol. The near complete overlap of the control and CD90 channels indicates that the resultant CD90-depleted CDC population contains little, if any, CD90⁺ cells. Figure 18D depicts white light microscopy images of the CD90-depleted CDCs. While growth is modestly more sparse (perhaps a result of the removal of cells and plating of a similar volume), the morphometry of the CD90-depleted CDCs is similar to non-depleted. This data suggests that, at least with respect to the growth and physical characteristic of the CDCs in culture, the lack of CD90 does not appear to adversely impact the CDCs.

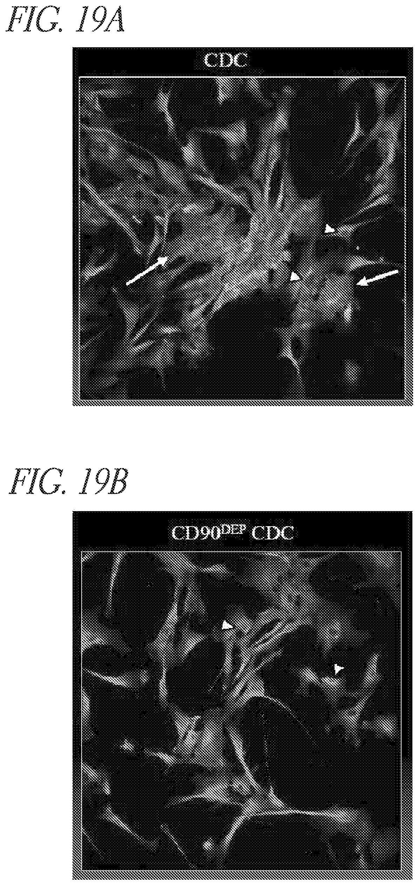

Figures 19A and 19B depict immunocytochemistry that indicates that the CD90 depletion protocol was specific to CD90. Figure 19A shows CD90 positive cells (arrows) that co-localize with CD105 positive CDCs (all cells in field are CD105 positive, arrowheads indicate cell nuclei). In contrast, Figure 19B shows the CDCs after CD90 depletion, where no cells are detected that express CD90 (cells shown are CD105 positive, arrowheads indicate cell nuclei).

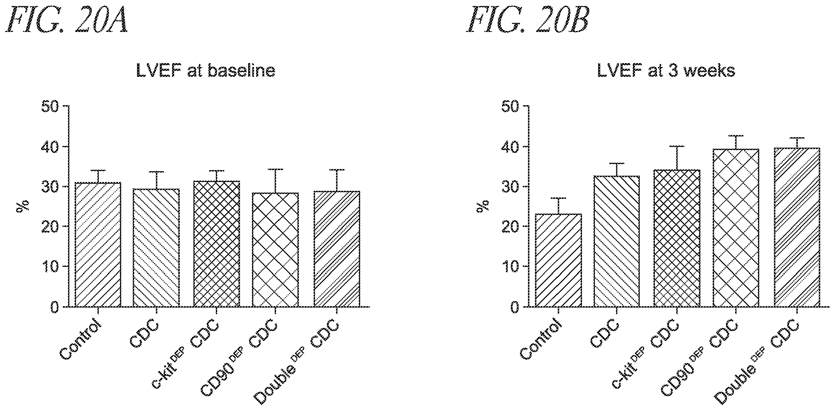

Figures 20A-20B depict functional data after administration of the indicated CDC populations to SCID mice having an myocardial infarction (using the model described in detail above). Cardiac function was assessed by measuring left ventricular ejection fraction (LEFV) using echocardiography at baseline (Figure 20A) and 3 weeks after CDC administration (Figure 20B). Figure 20A shows all the treatment groups having similar LVEF (-30%) at baseline, indicating that the effects of the induced MI were similar across all mice. After 3 weeks, however (Figure 20B), noticeable differences in LVEF were detected. The control mice at three weeks showed a decline in function (about 8% reduction as compared to baseline). Non-depleted CDCs (shown as "CDC") did not show any statistical improvement in function at three weeks post-administration, as compared to baseline, but resulted in significantly higher LVEF as compared to control hearts at 3 weeks post-administration. CDCs depleted of ckit (c-kit^{DEP} CDC) showed a slight improvement over CDCs, but the change was not statistically significant (p>0.05). Both CD90-depleted CDCs and ckit/CD90-depleted CDCs showed a significant increase in LVEF 3 weeks post-administration (increases of -10-12% over baseline and -15% over untreated controls at 3 weeks). No significant differences were detected between the CD90-depleted CDCs and ckit/CD90-depleted CDCs, with respect to LVEF. These data suggest, unexpectedly, that the absence of the CD90 subpopulation of CDCs leads to an increase in the therapeutic efficacy of the resulting CDCs. In several embodiments, therefore, CDCs depleted or substantially depleted of CD90 (and/or of ckit) are used in the treatment of damaged or diseased cardiac tissue. Substantially depleted shall be given its ordinary meaning and shall also include depleted by more than 90% (e.g., less than 10%, less than 5%, less than 2%, or 0% of the depleted subpopulation is present as compared, for example, to populations/cultures that are not processed according to embodiments described herein).

In experiments designed to replicate the post-hoc analysis of CD90 expression and therapeutic benefit in the CADUCEUS trial, five different CDC lines, each with differing levels of CD90 expression were evaluated in the SCID mouse MI model. The change in LVEF was assessed 3-weeks post-administration, as discussed above. Figure 22A shows the change in LVEF as compared to the five non-depleted CDC preparations, each showing their approximate CD90 expression level (-10%, -15%, -25%, -25%, -40%) and the CD90-depeleted CDCs (with less than 2% CD90 expression; shown far left). As with the CADUCEUS data, the greater the amount of CD90 present in the CDCs that were administered, the lesser the therapeutic benefit realized (at least by measurement of LVEF). This data is also represented in Figure 21B, which confirms a statistically significant negative correlation between CD90 and change in LVEF.

Anatomical data also indicate that CDCs depleted of at least one subpopulation (e.g., either ckit or CD90) or both subpopulations lead to increased therapeutic efficacy. Figure 22A shows Masson's Trichrome staining of three representative sections of control hearts (post-MI). The heart wall is noticeably thinned and has abundant scar tissue present. Figure 22B shows staining of mice treated with non-depleted CDCs. In comparison to the control hearts, the wall thickness appears substantially increased, and the Trichrome staining reveals an increased amount of viable cardiac tissue. Figure 23B depicts hearts from mice treated with CD90-depleted CDCs. As compared to control hearts and to those treated with non-depleted CDCs, heart walls from mice treated with CD90-depleted CDCs show a further increase in wall thickness as well as a further increase in viable tissue. Similarly, Figure 23D show sections of heart tissue from mice treated with double depleted CDCs (e.g., depleted of both CD90 and ckit subpopulations) and reveals increased wall thickness and increased viable tissue as compared to both control hearts and hearts treated with non-depleted CDCs. This anatomical data is summarized in Figure 22E and 22F (which depict infarct wall thickness and viable tissue, respectively). As shown in Figure 22E, administration of CDCs results in a significant increase in wall thickness. Further significant increases result when CD90-depleted CDCs or double depleted CDCs are administered (though no significant differences in wall thickness are detected between these two populations). Similarly, in Figure 22F, CDC administration results in a significant increase in viable tissue, with further significant increases in viable tissue resulting from administration of either CD90-depleted CDCs or double depleted CDCs.

Thus, in several embodiments, the administration of CD90-depeleted CDCs (in whole or in part) results, surprisingly, in an improved therapeutic efficacy, as measured by established functional cardiac assays. In several embodiments, additionally the CD90-depleted cell population leads to a regeneration of cardiac tissue and/or an increase in viable cardiac tissue. Likewise, administration of ckit and CD90-depeleted CDCs (in whole or in part) surprisingly results in an improved cardiac function. In several embodiments, additionally the ckit and CD90-depleted cell population leads improved cardiac tissue viability. While these data suggest that functional improvement and increased viability go hand-in-hand, depending on the embodiment, functional benefit may be realized without a corresponding increase in new and/or viable tissue, and vice versa. However, in several embodiments, the functional benefits are associated with both increased tissue function and increased tissue viability.

Experiments were next performed to determine the impacts of depletion of CD90 and/or ckit subpopulations on the ability of CDCs to promote cardiomyocyte proliferation. Neonatal rat cardiomyocytes (NRCM) were co-cultured with either CDCs (Figure 23A), CD90 depleted CDCs (23B), or doubly depleted CDCs (23C). Figures 23A-23C show immunocytochemistry staining of each of the co-cultures for ki67 (a marker of proliferation) and alpha sarcomeric actin (cardiac tissue marker). The percent of total proliferating myocytes (as measured by Ki67 positive staining) is shown in Figure 23D. These data indicate that depletion of the CD90 positive subpopulation and/or the depletion of both the CD90 positive subpopulation and the ckit positive subpopulation do not adversely affect the ability of cardiomyocytes to proliferate.

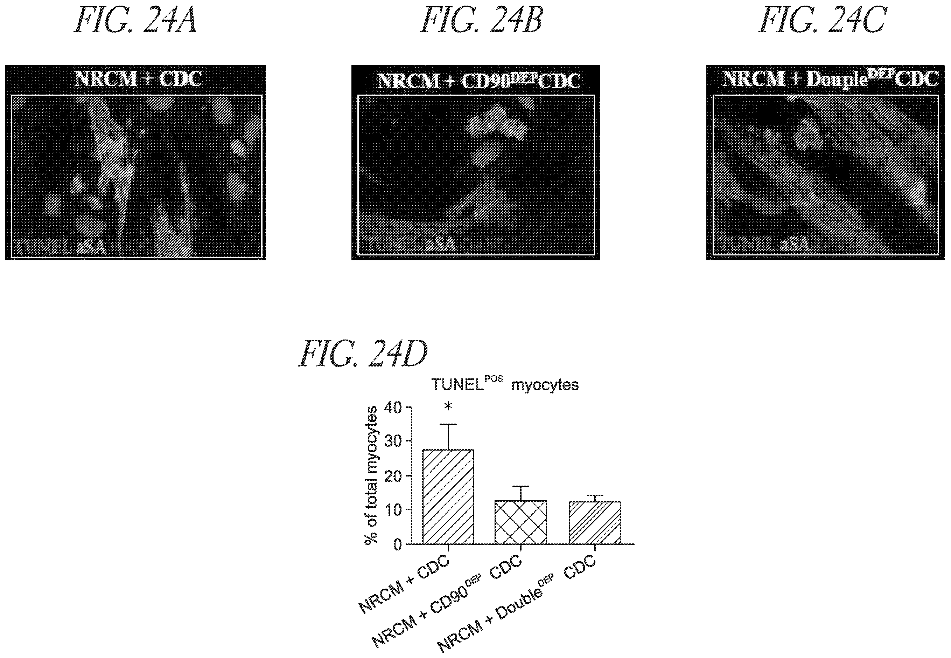

The impact of depletion of CD90 and/or ckit subpopulations on the apoptosis of cardiomyocytes was also assessed. NRCM were co-cultured with either CDCs (Figure 24A), CD90 depleted CDCs (24B), or doubly depleted CDCs (24C) and stained for TUNEL (marker of apoptosis) and alpha sarcomeric actin. Figure 24D summarizes the results and indicates that depletion of the CD90 subpopulation and depletion of both the CD90 positive subpopulation and the ckit positive subpopulation result in a significantly reduced rate of apoptosis (as compared to non-depleted CDCs) in NRCMs. Thus, in some embodiments, surprisingly, the removal of specific subpopulations results in an improved therapeutic efficacy, as manifest by a reduction in apoptosis that would occur, for example, after a myocardial infarction. This improved efficacy is associated with, in several embodiments, no adverse changes in the ability of CDCs to promote cardiomyocyte proliferation, which would lead to increases in the amount of viable cardiac tissue when the CDCs were administered post-MI.

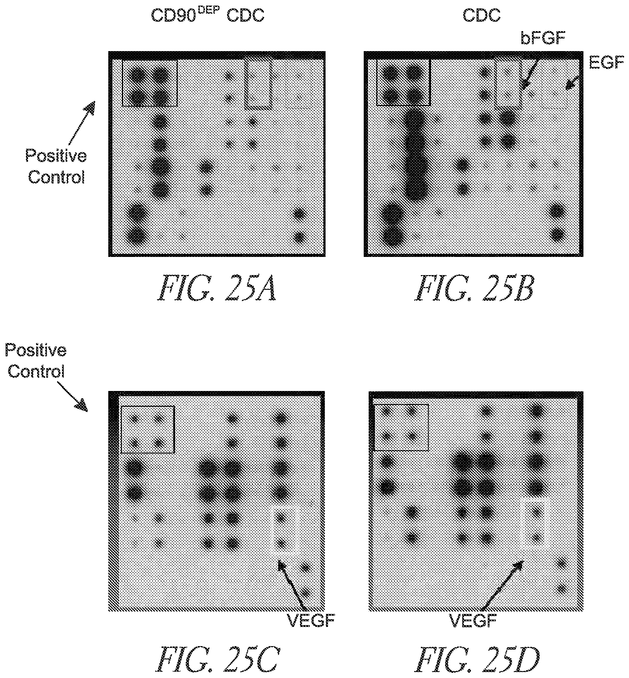

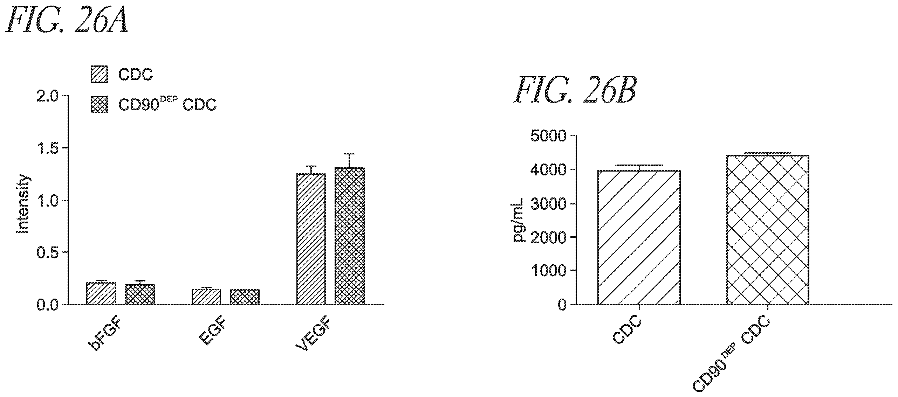

Cytokine arrays were used to evaluate various factors (e.g., cytokines, growth factors, paracrine factors) released by the CDCs and CDCs after specific depletion of subpopulations. The array analysis was performed by established commercial methods. Figures 25A-25D depict the results of a cytokine analysis for CD90-depleted CDCs (Panels A and C), as compared to non-depleted CDCs (Panels B and D). Figure 25A shows the array results and highlights the release (as evaluated by dot intensity) of basic fibroblast growth factor (bFGF), epidermal growth factor (EGF), and vascular endothelial growth factor (VEGF). Figure 26A is a histogram depicting the comparison of the release of each of these growth factors, but the two types of cells. As indicated in histogram, there appears to be no significant differences in release of bFGF, EGF, or VEGF between non-depleted CDCs and CDCs depleted of the CD90⁺ subpopulation. The results of the cytokine array were confirmed using an ELISA assay, shown in Figure 26B, which indicates no difference in the release of VEGF between the two CDC populations. Thus, the lack of a change in the release of bFGF, EGF, or VEGF when CDCs are depleted of the CD90⁺ subpopulation correlates with the maintenance of the ability of the CD90 depleted CDCs to promote proliferation of cardiomyocytes.

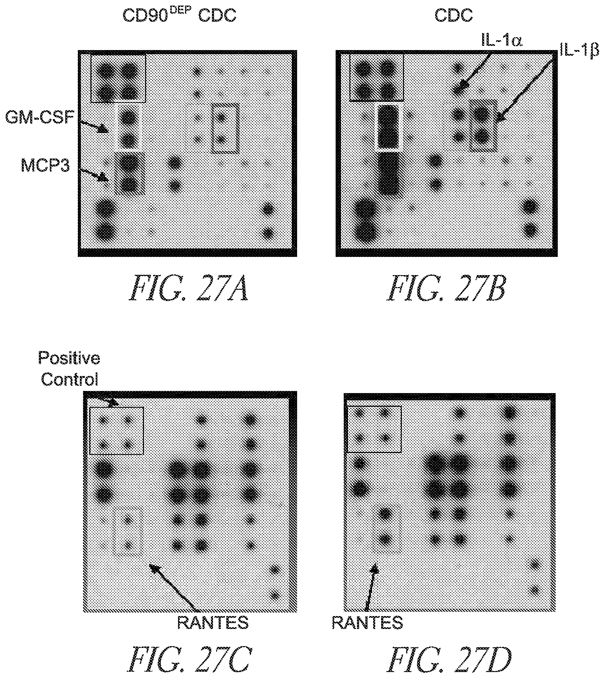

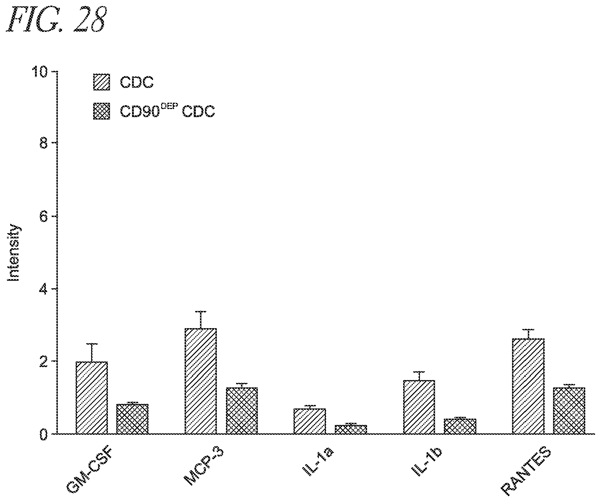

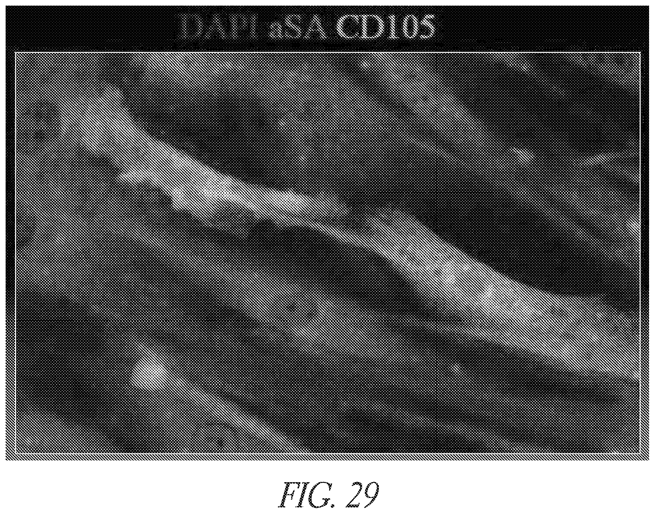

Figures 27A-27D depict an analysis specific for inflammatory cytokines, namely IL-1 alpha, IL-1 beta, monocyte chemotactic protein 3 (MCP3), granulocyte macrophage-colony stimulating factor (GM-CSF) and RANTES. IL-1 alpha and IL-1 beta are members of the interleukin-1 superfamily and play a role in the initiation of certain inflammatory responses. MCP3 30 in the recruitment of immune cells (particularly monocytes) to sites of inflammation and/or infection. GM-CSF is involved in the mobilization of granulocytes and monocytes from the bone marrow. RANTES plays an active role in the recruitment of leukocytes to sites of inflammation. In contrast to the growth factors discussed above, the depletion of the CD90⁺ subpopulation of CDCs results in an alteration of the release of these inflammatory cytokines. Figure 28 is a histogram summarizing the results of the inflammatory cytokine array. As shown, CD90 depleted CDCs release less GM-CSF, MCP3, 11-1 alpha, II-1 beta, and RANTES, as compared to non-depleted CDCs. As such, these data suggest that inflammatory responses may limit the therapeutic efficacy of CDCs and, coordinately that a reduction in the release of pro-inflammatory cytokines can improve the therapeutic efficacy. In several embodiments, the reduction in the release of inflammatory cytokines leads to a reduction in the number of immune cells being delivered or attracted to the cardiac tissue post-MI. The reduced influx of immune cells may, depending on the embodiment, be associated with a reduction in the apoptosis of the cardiac tissue. In conjunction with the reduced cell death, the CD90 depleted CDCs are unchanged with respect to their ability to promote proliferation of cardiomyocytes. As a result, the administration of CD90 depleted CDCs, in several embodiments, results in less cell die off and maintained cell proliferation, which in turn increases the amount of viable cardiac tissue post-MI. With this greater amount of viable cardiac tissue the cardiac function can be maintained and/or improved as a result. Figure 29 shows the results of culturing a population of doubly depleted CDCs, which showed the ability to differentiate into cardiomyocytes that expressed alpha sarcomeric actin. This ability may again be tied the ability of depleted CDC populations to increase viable cardiac tissue mass.

Thus, in several embodiments, CD90 depleted CDCs are used in the repair or regeneration of cardiac tissue. In several embodiments, doubly depleted CDCs are used (e.g., those depleted of both CD90 and ckit subpopulations). In several embodiments, the CDCs administered to repair and/or regenerate cardiac tissue and realize improved cardiac function and/or, increased cardiac tissue viability are CD105^{POS}/CD90^{NEG}/ckit^{NEG}. The depletion of certain specific subpopulations, as demonstrated herein, not only has the potential to improve the therapeutic efficacy of the resulting depleted population of CDCs, but may provide certain advantages as the cell population becomes more uniform (e.g., reduced adverse immune responses from the recipient, more well-characterized potency profile, etc.). However, in some embodiments, non-depleted CDCs may also be used. In several embodiments, for example those in which multiple doses are given over time, the doses may comprise alternating between non-depleted CDCs and CDCs depleted of one or more subpopulations.

Any methods disclosed herein need not be performed in the order recited. The methods disclosed herein include certain actions taken by a practitioner; however, they can also include any third-party instruction of those actions, either expressly or by implication. For example, actions such as "administering a population of therapeutic cells depleted of cells expressing a marker" include "instructing the administration of a population of therapeutic cells depleted of cells expressing a marker."

The ranges disclosed herein also encompass any and all overlap, subranges, and combinations thereof. Language such as "up to," "at least," "greater than," "less than," "between," and the like includes the number recited. Numbers preceded by a term such as "about" or "approximately" include the recited numbers. For example, "about 10 nanometers" includes "10 nanometers."

## Claims

1. A composition comprising a population of cardiosphere-derived cells (CDCs) depleted of at least 90%, 95% or 99% of cells expressing the CD90 marker, wherein said population of CDCs are obtained from non-embryonic donor tissue that is subjected to selective depletion.

2. The composition of Claim 1, wherein said population of CDCs is entirely depleted of cells expressing the CD90 marker.

3. The composition of Claim 1, wherein said population of CDCs is further depleted of at least 90%, 95% or 99% of cells expressing the c-kit stem cell marker.

4. The composition according to any one of the preceding Claims, wherein the CDCs are 5 to 35 µm (microns) in diameter and are suitable for systemic delivery to a recipient in need of repair of damaged or diseased cardiac tissue.

5. The composition according to any one of the preceding Claims, wherein the therapeutic population of cells further comprises endothelial cells.

6. The composition of Claim 5, wherein said endothelial cells express at least one of the following endothelial markers: CD105, KDR, flk-1, CD31, von Willebrand factor, Ve-cadherin and smooth muscle alpha actin.

7. The composition according to any one of the preceding Claims for use in the repair of damaged or diseased cardiac tissue.

8. The composition for use according to Claim 7, wherein the composition is administered to a subject having damaged or diseased cardiac tissue in between 1 x 10⁶ and 100 x 10⁶ cells.

9. The composition for use according to Claim 7 or 8, wherein the composition can improve the function and/or viability of said damaged cardiac tissue.

## Patentansprüche

1. Zusammensetzung, die eine Population von Kardiosphären-abgeleiteten Zellen (CDCs) umfasst, die von mindestens 90 %, 95 % oder 99 % von Zellen, die den CD90-Marker exprimieren, depletiert sind, wobei die Population von CDCs aus nichtembryonischem Spendergewebe erhalten wird, das einer selektiven Depletion unterzogen wird.

2. Zusammensetzung nach Anspruch 1, wobei die Population von CDCs vollständig von Zellen, die den CD90-Marker exprimieren, depletiert ist.

3. Zusammensetzung nach Anspruch 1, wobei die Population von CDCs weiter von mindestens 90 %, 95 % oder 99 % von Zellen, die den c-kit-Stammzell-Marker exprimieren, depletiert ist.

4. Zusammensetzung nach einem der vorstehenden Ansprüche, wobei die CDCs 5 bis 35 µm (Mikron) im Durchmesser sind und für die systemische Abgabe an einen Empfänger geeignet sind, der eine Reparatur von geschädigtem oder erkranktem Herzgewebe benötigt.

5. Zusammensetzung nach einem der vorstehenden Ansprüche, wobei die therapeutische Population von Zellen weiter Endothelzellen umfasst.

6. Zusammensetzung nach Anspruch 5, wobei die Endothelzellen mindestens einen der folgenden Endothelmarker exprimieren: CD105, KDR, flk-1, CD31, von-Willebrand-Faktor, VE-Cadherin und Alpha-Glattmuskel-Aktin.

7. Zusammensetzung nach einem der vorstehenden Ansprüche für die Verwendung bei der Reparatur von geschädigtem oder erkranktem Herzgewebe.

8. Zusammensetzung für die Verwendung nach Anspruch 7, wobei die Zusammensetzung von zwischen 1 x 10⁶ und 100 x 10⁶ Zellen an ein Subjekt verabreicht wird, das geschädigtes oder erkranktes Herzgewebe aufweist.

9. Zusammensetzung für die Verwendung nach Anspruch 7 oder 8, wobei die Zusammensetzung die Funktion und/oder Viabilität des geschädigten Herzgewebes verbessern kann.

## Revendications

1. Composition comprenant une population de cellules dérivées de la cardiosphère (CDC) ayant un appauvrissement d'au moins 90%, 95% ou 99% des cellules exprimant le marqueur CD90, dans laquelle ladite population de CDC est obtenue à partir d'un tissu de donneur non embryonnaire qui est soumis à un appauvrissement sélectif.

2. Composition selon la revendication 1, dans laquelle ladite population de CDC est entièrement appauvrie en cellules exprimant le marqueur CD90.

3. Composition selon la revendication 1, dans laquelle ladite population de CDC possède en outre un appauvrissement d'au moins 90%, 95% ou 99% des cellules exprimant le marqueur de cellules souches c-kit.

4. Composition selon l'une quelconque des revendications précédentes, dans laquelle les CDC possèdent un diamètre allant de 5 à 35 µm (microns) et sont convenables pour une administration systémique à un receveur ayant besoin d'une réparation de tissu cardiaque endommagé ou malade.

5. Composition selon l'une quelconque des revendications précédentes, dans laquelle la population thérapeutique de cellules comprend en outre des cellules endothéliales.

6. Composition selon la revendication 5, dans laquelle lesdites cellules endothéliales expriment au moins l'un parmi les marqueurs endothéliaux suivants : CD 105, KDR, flk-1, CD31, le facteur de von Willebrand, la VE-cadhérine, et l'alpha-actine des muscles lisses.

7. Composition selon l'une quelconque des revendications précédentes, pour une utilisation dans la réparation de tissu cardiaque endommagé ou malade.

8. Composition pour une utilisation selon la revendication 7, où la composition est administrée à un sujet ayant un tissu cardiaque endommagé ou malade à entre 1 x 10⁶ et 100×10⁶ cellules.

9. Composition pour une utilisation selon la revendication 7 ou 8, où la composition peut améliorer la fonction et/ou la viabilité dudit tissu cardiaque endommagé.
